(19) Europäisches Patentamt
European Patent Office
Office européen des brevets

(11) Veröffentlichungsnummer: **0 345 635 B1**

(12) # EUROPÄISCHE PATENTSCHRIFT

(45) Veröffentlichungstag der Patentschrift: **05.10.94**

(51) Int. Cl.⁵: **C07D 413/04**, C07D 409/04, C07D 405/04, C07D 407/04, A01N 43/16, A01N 43/56

(21) Anmeldenummer: **89109911.1**

(22) Anmeldetag: **01.06.89**

Die Akte enthält technische Angaben, die nach dem Eingang der Anmeldung eingereicht wurden und die nicht in dieser Patentschrift enthalten sind.

(54) **Herbizide Tetrahydropyran-2,4-dione.**

(30) Priorität: **07.06.88 DE 3819347**

(43) Veröffentlichungstag der Anmeldung:
**13.12.89 Patentblatt 89/50**

(45) Bekanntmachung des Hinweises auf die Patenterteilung:
**05.10.94 Patentblatt 94/40**

(84) Benannte Vertragsstaaten:
**BE CH DE FR GB IT LI NL**

(56) Entgegenhaltungen:
**EP-B- 0 012 158**
**WO-A-88/04652**
**DE-A- 3 701 298**
**DE-C- 2 524 577**
**GB-A- 2 140 803**

(73) Patentinhaber: **BASF Aktiengesellschaft**
**Carl-Bosch-Strasse 38**
**D-67063 Ludwigshafen (DE)**

(72) Erfinder: **Kast, Juergen, Dr.**
**Kastanienstrasse 24**
**D-6737 Boehl-Iggelheim (DE)**
Erfinder: **Kolassa, Dieter, Dr.**
**Moltkestrasse 8**
**D-6700 Ludwigshafen (DE)**
Erfinder: **Meyer, Norbert, Dr.**
**Dossenheimer Weg 22**
**D-6802 Ladenburg (DE)**
Erfinder: **Wuerzer, Bruno, Dr.**
**Ruedigerstrasse 13**
**D-6701 Otterstadt (DE)**
Erfinder: **Westphalen, Karl-Otto, Dr.**
**Mausbergweg 58**
**D-6720 Speyer (DE)**
Erfinder: **Rademacher, Wilhelm, Dr.**
**Ausstrasse 1**
**D-6703 Limburgerhof (DE)**
Erfinder: **Jung, Johann, Prof.-Dr.**
**Hardenburgstrasse 19**
**D-6703 Limburgerhof (DE)**

EP 0 345 635 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Tetrahydropyran-2,4-dione der Formel I,

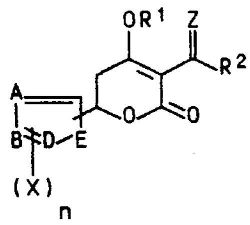

I

in der die Substituenten und der Index folgende Bedeutung haben

$R^1$

Wasserstoff;

$R^2$

$C_1$-$C_6$-Alkyl;

A, B, D

=CH- oder =N-, wobei mindestens eine der Variablen für Stickstoff steht;

E

Sauerstoff, Schwefel oder -$NR^7$-, wobei

$R^7$

für $C_1$-$C_6$-Alkyl, Benzyl oder Phenyl, welches ein- bis dreifach durch Halogen und/oder $C_1$-$C_6$-Halogenalkyl substituiert sein kann, steht.

X

Halogen, $C_1$-$C_6$-Alkyl oder $C_3$-$C_6$-Cycloalkyl, welche ein- bis dreifach durch Halogen, $C_1$-$C_6$-Alkoxy und/oder $C_1$-$C_6$-Alkylthio substituiert sein können; $C_2$-$C_6$-Alkenyl; $C_2$-$C_6$-Alkinyl; $C_1$-$C_6$-Alkoxy; $C_2$-$C_6$-Alkenyloxy; $C_1$-$C_6$-Alkylthio; $C_1$-$C_6$-Alkoxycarbonyl; $C_2$-$C_6$-Alkanoyloxy; $NR^5R^6$; Phenyl, welches ein- bis dreifach durch Halogen, Nitro, Cyano, $C_1$-$C_6$-Alkyl, $C_1$- $C_6$-Alkoxy, $C_1$-$C_6$-Halogenalkyl und/oder $NR^3R^4$ substituiert sein kann;

n

0, 1 oder 2, wobei die Reste X im Falle von n = 2 auch verschieden sein können;

$R^3$, $R^4$, $R^5$, $R^6$

Wasserstoff, $C_1$-$C_6$-Alkyl;

Z

Sauerstoff oder ein Oximether-Rest -$NOR^8$, wobei

$R^8$ für

$C_1$-$C_6$-Alkyl oder $C_2$-$C_6$-Alkenyl, welche ein- bis dreifach durch Halogen, $C_1$-$C_6$-Alkoxy und/oder $C_1$-$C_6$-Alkylthio substituiert sein können; $C_2$-$C_6$-Alkinyl; $C_2$-$C_6$-Halogenalkinyl; $C_1$-$C_6$-Alkyl oder $C_2$-$C_6$-Alkenyl, jeweils substituiert bis dreifach durch Phenyl, welches ein- bis dreifach durch Halogen, Nitro, Cyano, $C_1$-$C_6$-Alkyl, $C_1$-$C_6$-Halogenalkyl, $C_1$-$C_6$-Alkoxy und/oder $C_1$-$C_6$-Alkylthio substituiert sein kann; Thenyl, welches ein- bis dreifach durch Halogen substituiert sein kann, steht,

sowie deren umweltverträglichen Salze.

Außerdem betrifft die Erfindung die Herstellung der Verbindungen I, deren Verwendung als Herbizide sowie als Wachstumsregulatoren und entsprechende Mittel.

Die Verbindungen I können in mehreren tautomeren Formen vorliegen, die aber vom Patentanspruch umfaßt werden.

Es ist bekannt, daß Tetrahydropyran-2,4-dionoximetherderivate eine herbizide Wirkung gegenüber monokotylen Pflanzen aufweisen. Beispiele hierfür sind Tetrahydropyran-2,4-dione, die in 6-Position einen Phenylrest tragen (GB-A-21 40 803).

Es werden jedoch Verbindungen gesucht, die bei geringeren Aufwandmengen eine bessere Verträglichkeit mit Kulturpflanzen (Selektivität) bei guter Wirkung gegen Schadpflanzen aufweisen.

Entsprechend dieser Aufgabe wurden die eingangs definierten Tetrahydropyran-2,4-dionderivate gefunden.

Ferner wurde gefunden, daß Derivate der Formel I, in denen Z eine Oximethergruppe (-$NOR^8$) bedeutet, eine gute herbizide Wirkung besitzen und Derivate, in denen Z Sauerstoff bedeutet, wachstumsregulierende Eigenschaften haben.

Die Tetrahydropyran-2,4-dionderivate der Formel I, in denen Z den Rest -NOR$^8$ bedeutet, können durch Umsetzung derjenigen Tetrahydropyran-2,4-dionderivate der Formel I, in denen Z Sauerstoff bedeutet, mit einem entsprechenden Hydroxylamin oder seiner Ammoniumverbindung $R^8ONH_2$ bzw. $R^8ONH_3^{\oplus}W^{\ominus}$ erhalten werden. Dabei steht $W^{\ominus}$ für ein Anion einer anorganischen Säure wie Chlorid, Bromid, Jodid, Hydrogensulfat und Phosphat; die Umsetzungstemperatur liegt im allgemeinen bei oberhalb von 0°C bis gegen 80°C.

Zweckmäßigerweise und bevorzugt setzt man das Tetrahydropyran-2,4-dionderivat I, in dem Z Sauerstoff bedeutet, mit der Ammoniumverbindung in Gegenwart einer festen Base in einem Lösungsmittel bei einer Temperatur von 20°C bis 80°C um. Geeignete Basen sind z.B. Carbonate, Hydrogencarbonate, Acetate, Alkoholate, Hydroxyde und Oxide von Alkali und Erdalkalimetallen, insbesondere Natriumhydroxid, Kaliumhydroxid, Magnesiumoxid, Calciumoxid. Außerdem können organische Basen, wie Pyridin oder tertiäre Amine, Verwendung finden. Die Base wird in stöchiometrischer Menge oder einem Überschuß zugesetzt.

Als Lösungsmittel sind beispielsweise Dimethylsulfoxid; Alkohole, wie Methanol, Ethanol oder Isopropanol; Toluol; Hexan oder Cyclohexan; chlorierte Kohlenwasserstoffe, wie Chloroform, Dichlormethan; Ester, wie Essigsäureethylester; oder Ether wie Dioxan oder Tetrahydrofuran, geeignet.

Die Tetrahydropyran-2,4-dione der Formel I, in denen Z Sauerstoff bedeutet, erhält man in an sich bekannter Weise durch Umsetzung eines entsprechenden Tetrahydropyran-2,4-dionderivats der Formel II

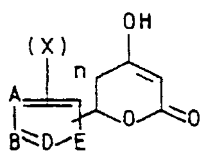

II

mit einem Säurechlorid, durch das der Substituent R$^2$ eingeführt wird, in Gegenwart einer Base (z.B. Triethylamin) in einem Verdünnungsmittel (z.B. Tetrahydrofuran) zum Enolester und anschließend mit einem sauren oder basischen Katalysator im gleichen oder in einem anderen Lösungsmittel (z.B. Essigester, Toluol) bei einer Temperatur zwischen 0 und 100°C. Beispiele für saure oder basische Katalysatoren sind Aluminiumchlorid, Eisenchlorid, Imidazolderivate, z.B. N-Methylimidazol bzw. ein Pyridin, z.B. Dimethylaminopyridin.

Zur Herstellung der Verbindungen der Formel II gibt es mehrere bekannte Verfahren, die stets von einem Aldehyd

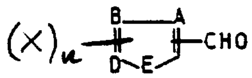

ausgehen:

a) Umsetzung mit einem entsprechenden 4-Brom-3-methoxy-crotonsäureester in einem Lösungsmittel wie Ether, Tetrahydrofuran, Benzol in Gegenwart von Zink (J. Heterocyclic Chem. 21, 1755 (1984)) bzw.

b) Umsetzung mit Diketen in einem Lösungsmittel wie Dichlormethan mit einer Lewissäure (z.B. Titantetrachlorid) als Katalysator (Chem. Letters, 1975, 101), oder

c) Umsetzung mit dem Dianion von Acetessigsäurealkylester, d.h. z.B. mit dem Di-natriumsalz in einem Lösungsmittel wie Tetrahydrofuran (Angew. Chem. 86, 40 (1974)).

Zum Aldehyd gelangt man z.B. durch Oxidation eines entsprechenden Alkohols, Reduktion von Carbonsäurederivaten oder elektrophile Aromatensubstitution wie z.B. Vilsmeyer-Reaktion.

Die Wirkungsrichtung der erfindungsgemäßen Tetrahydropyran-2,4-dionderivate wird durch das Substitutionsmuster beeinflußt; z.B. besitzen die Verbindungen, bei denen Z für den Rest N-OR$^8$ steht, eine gute herbizide Wirkung, während die Tetrahydropyran-2,4-dionderivate, in denen Z Sauerstoff bedeutet, vorzugsweise wachstumsregulierende Eigenschaften aufweisen.

Die Verbindungen der Formel I Können in mehreren, formal von der Formel I verschiedenen tautomeren Formen auftreten, die ebenfalls zur Erfindung gehören.

Für den Fall, daß olefinisch ungesättigte Substituenten vorhanden sind, können in bekannter Weise E- und Z-Isomere auftreten, die gleichermaßen zur Erfindung gehören.

In den Fällen, in denen $R^8$ Halogenalkyl oder Halogenalkenyl bedeutet, sind diejenigen mit einem bis drei Halogenatomen besonders bevorzugt.

Zwecks besserer Übersicht sind die erfindungsgemäßen Verbindungen nachstehend in Gruppen aufgeführt:

Oxazole, Thiazole und Imidazole der Formel I.1 bis I.9

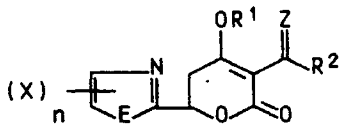

I.1 E = O
I.2 E = S
I.3 E = $NR^7$

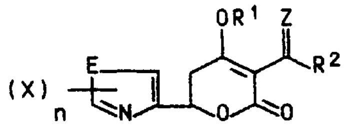

I.4 E = O
I.5 E = S
I.6 E = $NR^7$

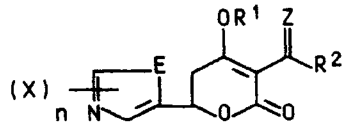

I.7 E = O
I.8 E = S
I.9 E = $NR^7$

Isoxazole, Isothiazole und Pyrazole der Formeln I.10 bis I.18

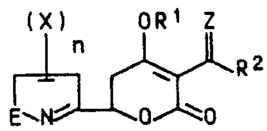

I.10 E = O
I.11 E = S
I.12 E = $NR^7$

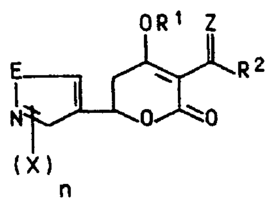

I.13 E = O
I.14 E = S
I.15 E = $NR^7$

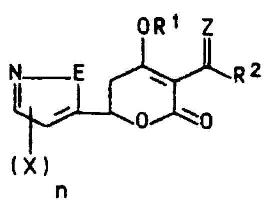

I.16 E = O
I.17 E = S
I.18 E = $NR^7$

1,2,3-Oxadiazole, 1,2,3-Thiadiazole und 1,2,3-Triazole der Formeln I.19 bis I.24

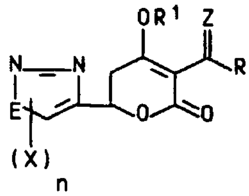

I.19 E = O
I.20 E = S
I.21 E = $NR^7$

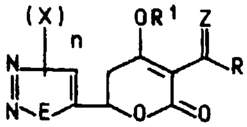

I.22 E = O
I.23 E = S
I.24 E = $NR^7$

1,2,4-Oxadiazole, 1,2,4-Thiadiazole und 1,2,4-Triazole der Formeln I.25 bis I.30

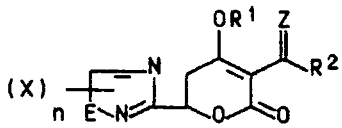

I.25 E = O
I.26 E = S
I.27 E = $NR^7$

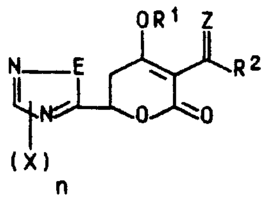

I.28 E = O
I.29 E = S
I.30 E = $NR^7$

1,3,4-Oxadiazol, 1,3,4-Thiadiazol der Formeln I.31 bis I.33

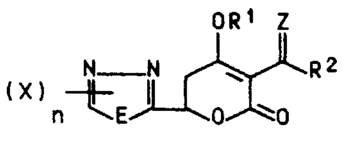

I.31 E = O
I.32 E = S
I.33 E = $NR^7$

$R^1$ in Formel I steht für Wasserstoff, weiterhin für ein einwertiges Metallkation, insbesondere Natrium, Kalium, oder ein Äquivalent eines mehrwertigen Kations, z.B. ein Erdalkalimetallkation, insbesondere Magnesium, Calcium und andere landwirtschaftlich brauchbare Kationen wie Mangan-, Kupfer-, Zink- und Eisenkation sowie Ammonium-, Phosphonium, Sulfonium-und Sulfoxoniumkation wie Ammonium, Tetraalkylammonium, Benzyltrialkylammonium, Trialkylsulfonium oder Trialkylsulfoxonium.

$R^2$ in Formel I steht für einen unverzweigten, verzweigten oder cyclischen Alkylrest von 1 bis 6 Kohlenstoffatomen, z.B. für Methyl, Ethyl, n-Propyl, i-Propyl, n-Butyl, s-Butyl, i-Butyl, t-Butyl, n-Pentyl, i-Pentyl, s-Pentyl, t-Pentyl, neo-Pentyl, n-Hexyl, Cyclopropyl, Cyclobutyl, Cyclopentyl und Cyclohexyl.

Besonders bevorzugt sind unverzweigte Alkylreste von 1 bis 4 Kohlenstoffatomen, d.h. Methyl, Ethyl, Propyl, Butyl; insbesondere Ethyl und Propyl.

Bevorzugt sind von den Ringgliedern A, B, D und E nicht mehr als drei gleichzeitig Heteroatome.

X bedeutet bevorzugt Fluor, Chlor, Brom, Methyl, Ethyl, n-Propyl, Isopropyl, n-Butyl, i-Butyl, s-Butyl, t-Butyl, n-Pentyl, Cyclopropyl, Cyclobutyl, Cyclopentyl, Cyclohexyl, Methoxy, Ethoxy, i-Propyloxy, t-Butyloxy, Methylthio, Ethylthio, Dimethylamino, Diethylamino, Dibutylamino, Methoxymethyl, Ethoxymethyl, Methylthiomethyl, Ethylthiomethyl, 1-Methoxyethyl, 2-Methoxythio-ethyl, 1,3-Dimethoxypropyl, 2-Ethoxyethyl, 1-Methyl-2-methylthio-ethyl, 2-Methyl-1-methylthiomethylpropyl, 1-Methyl-butyl, 2-Methyl-1-propenyl, Phenyl, 4-Methylphenyl, 4-Trifluormethylphenyl, 2,4-Dichlorphenyl, 4-Methoxyphenyl, 2,4,6-Trimethylphenyl, 3,5-Dibromphenyl, 4-Methylthiophenyl, 4-Fluorphenyl; insbesondere Methyl, Ethyl, n-Propyl, Isopropyl, s-Butyl, t-Butyl, Cyclopropyl, Cyclopentyl, 1-Methoxyethyl, Dimethylamino, 4-Fluorphenyl, Chlor.

$R^8$ bedeutet bevorzugt Methyl, Ethyl, Propyl, Allyl, Prop-2-inyl, 3-Fluorpropyl, trans-3-Chlor-prop-2-enyl, trans-But-2-enyl, cis-3-Chlor-prop-2-enyl, 2-Chlor-prop-2-enyl, But-2-inyl, 2-Methoxyethyl, E-4(4-Fluorphenyl)but-2-enyl; E-4(4-chlorphenyl)but-2-enyl, E-4(4-tert. butylphenyl)but-2-enyl; E-4(4-Trifluorphenyl)but-2-enyl; E-4-Phenyl-but-2-enyl, Thenyl, 5-Thenyl, insbesondere Methyl, Ethyl, Propyl, Allyl, trans-3-chlor-prop-2-enyl, trans-But-2-enyl, 5-Chlor-thenyl.

n ist 0, 1 oder 2

$R^7$ bedeutet bevorzugt Methyl, Ethyl, Benzyl, Phenyl, Isopropyl, 4-Methoxyphenyl.

Bevorzugte Heterocyclen in 6-Position des Tetrahydropyran-2,4-dion-systems der Formel I sind Isoxazol-5-yl, Isoxazol-4-yl, Isoxazol-3-yl,

Isothiazol-5-yl, Isothiazol-4-yl, Isothiazol-3-yl, Pyrrazol-5-yl, Pyrrazol-4-yl, Pyrrazol-3-yl, 1,3-Oxazol-2-yl, 1,3-Oxazol-4-yl, 1,3-Oxazol-5-yl, 1,3-Thiazol-2-yl, 1,3-Thiazol-4-yl, 1,3-Thiazol-5-yl, Imidazol-2-yl, Imidazol-4-yl, Imidazol-5-yl, 1,2,3-Oxadiazol-4-yl, 1,2,3-Oxadiazol-5-yl, 1,2,3-Thiadiazol-4-yl, 1,2,3-Thiadiazol-5-yl,1,2,3-Triazol-4-yl, 1,2,3-Triazol-5-yl, 1,2,4-Oxadiazol-3-yl, 1,2,4-Oxadiazol-5-yl, 1,2,4-Thiadiazol-3-yl, 1,2,4,-Thiadiazol-5-yl, 1,2,4-Triazol-3-yl, 1,2,4-Triazol-5-yl, 1,3,4-Oxadiazol-2-yl, 1,3,4-Thiadiazol-2-yl, 1,3,4-Triazol-2-yl. Besonders bevorzugt sind Isoxazol-5-yl, Isoxazol-3-yl, Isothiazol-5-yl, 1,3-Thiazol-4-yl; 1,3-Thiazol-5-yl, Imidazol-2-yl, insbesondere Isoxazol-5-yl, Isoxazol-3-yl.

Als herbizide Wirkstoffe eignen sich bevorzugt die Verbindungen I, in denen Z eine Oximether-Gruppe ($-NOR^8$) bedeutet. Im Hinblick auf die wachstumsregulierende Wirkung sind diejenigen Verbindungen I bevorzugt, in denen Z Sauerstoff bedeutet. Die neuen Tetrahydropyran-2,4-dione I bzw. die sie enthaltenden herbiziden und wachstumsregulierenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 4.1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 4.4 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 4.28. werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 5.2 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 5.4 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 6.3 werden mit 97 Gewichtsteilen feinteiligem Kaolin vermischt. Man erhält auf diese Weise ein Stäubemittel, das 3 Gew.% des Wirkstoffs enthält.

VII. 20 Gewichtsteile des Wirkstoffs Nr. 8.2 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden bzw. wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an herbizidem Wirkstoff betragen je nach Bekämpfungsziel, Jahreszeit, Zielpflanzen und Wachstumsstadium 0,01 bis 3, vorzugsweise 0,05 bis 1,0 kg/ha aktive Substanz (a.S.).

Die Tetrahydropyran-2,4-dione der allgemeinen Formel I, in denen Z vorzugsweise Sauerstoff bedeutet, haben im allgemeinen sehr gute wachstumsregulierende Wirkung. Sie können praktisch alle Entwicklungsstadien einer Pflanze verschiedenartig beeinflussen und werden deshalb auch als Wachstumsregulatoren eingesetzt. Die Wirkungsvielfalt der Verbindungen hängt von verschiedenen Faktoren ab. Maßgeblich sind dabei vor allem

a) Pflanzenart und -sorte,

b) Zeitpunkt der Applikation, bezüglich Entwicklungsstadium der Pflanze und Jahreszeit,

c) Applikationsort und -verfahren (Samenbeize, Bodenbehandlung oder Blattapplikation)

d) klimatische Faktoren, z.B. Temperatur, Niederschlagsmenge, außerdem auch Tageslänge und Lichtintensität

e) Bodenbeschaffenheit (einschließlich Düngung),

f) Formulierung bzw. Anwendungsform des Wirkstoffs und

g) Aufwandmenge und Wirkstoffgehalt der Mittel.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, sind nachstehend einige Beispiele aufgeführt.

A. Mit den wachstumsregulierenden Tetrahydropyran-2,4-dionen I (Z = O) läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Bei Obstgehölzen und anderen Bäumen oder Sträuchern können dadurch kostenintensive Schnittmaßnahmen reduziert werden.

Von wirtschaftlichem Interesse ist auch die Erhöhung der Standfestigkeit von lageranfälligen Kulturen wie Getreide, Reis, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanzen ermöglicht.

Durch Anwendung der wachstumgsregulierenden Verbindungen I kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit den wachstumsregulierenden Verbindungen I läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse -dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den wachstumsregulierenden Mitteln auf der Basis von Tetrahydropyran-2,4-dionen I (Z = O) lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Die Tetrahydropyran-2,4-dione der Formel I (Z = O) können Ertragssteigerungen durch Eingriffe in den pflanzlichen Stoffwechsel bzw. durch Förderung oder Hemmung des vegetativen und/oder des generativen Wachstums verursachen.

C. Mit den Tetrahydropyran-2,4-dionen I (Z = O) lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Die Förderung der Ausbildung eines Trenngewebes zwischen der Blatt- und Sproßachse ist auch für ein gut kontrollierbares Entblättern von Nutzpflanzen z.B. Baumwolle wesentlich.

D. Mit den Tetrahydropyran-2,4-dionen I (Z = O) kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird

- das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie durch Spritzung über das Blatt zugeführt werden.

Infolge der hohen Verträglichkeit der Pflanzen für die wachstumsregulierenden Verbindungen I, in denen Z vorzugsweise Sauerstoff bedeutet, kann die Aufwandmenge stark variiert werden. Bei der Saatgutbehandlung werden im allgemeinen Wirkstoffmengen von 0,001 bis 500 g je Kilogramm Saatgut, vorzugsweise 0,01 bis 10 g, benötigt. Für die Blatt- und Bodenbehandlung sind im allgemeinen Gaben von 0,01 bis 10 kg/ha, bevorzugt 0,1 bis 5 kg/ha ausreichend.

In Anbetracht der Vielseitigkeit der Applikationsmethoden können die erfindungsgemäßen herbiziden und wachstumsregulierenden Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |

| Botanischer Name | Deutscher Name |
|---|---|
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Tetrahydropyran-2,4-dione I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile,

Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt. Verbindungen ohne diese Angaben lassen aufgrund ihrer strukturellen Ähnlichkeit eine entsprechende biologische Wirkung erwarten. Die wachstumsregulierenden Verbindungen I, in denen Z Sauerstoff bedeutet, entsprechen in ihren Resten den in der Tabelle aufgeführten herbiziden Verbindungen I.

Herstellungsbeispiel

a) 6-(3-Isopropylisoxazol-5-yl)-tetrahydropyran-2,4-dion

16,5 g Natriumhydrid in 350 ml THF wurden bei -10°C vorgelegt und 61,5 g Acetessigsäuremethylester zugetropft. Das Gemisch wurde auf -60°C abgekühlt und mit 344 ml 1,6-molarer n-Butyllithiumlösung in Hexan versetzt. Nach beendeter Zugabe wurde 30 min bei -60°C nachgerührt und dann 69,5 g 5-Formyl-3-isopropylisoxazol in 50 ml THF rasch bei -60°C zugetropft. Es wurde 30 min nachgerührt und dann 10 ml Methanol und anschließend 30 g Essigsäure zugegeben. Nach 10 min wurde auf 500 g Eis gegossen, auf Raumtemperatur erwärmt und 1 h nachgerührt. Die wäßrige Phase wurde dreimal mit Methylenchlorid extrahiert, unter Kühlung mit verd. Salzsäure auf pH 4,5 gebracht und mit Methylenchlorid extrahiert. Der Extrakt wird mit 5 %iger Natriumhydrogencarbonatlösung und Wasser gewaschen, mit Natriumsulfat getrocknet und im Rotationsverdampfer das Lösungsmittel abgezogen. Der Rückstand wird durch Verrühren mit Methyl-tert.-butylether kristallisiert und abgesaugt. Es werden 15 g reines Produkt erhalten.

b) 3-Butyryl-6-(3-Isopropylisoxazol-5-yl)-tetrahydropayran-2,4-dion

27,9 g 6-(3-Isopropylisoxazol-5-yl)-tetrahydropyran-2,4-dion wurden in 200 ml THF vorgelegt und mit 12,6 g Triethylamin versetzt. Bei 0°C wurden 13,3 g Buttersäurechlorid eingetropft und eine Stunde bei 0°C nachgerührt. Es wurde eingedampft, zwischen Methylenchlorid und Wasser verteilt. Die organische Phase wurde aufgearbeitet, d.h. mit Wasser, 10%iger Natriumcarbonatlösung und nochmals mit Wasser extrahiert, mit Natriumsulfat getrocknet und wieder eingedampft. Der Rückstand wird in trockenem Methylenchlorid gelöst und mit 1,3 g 4-(N,N-Dimethylamino)-pyridin 48 h gerührt. Nach dem Entfernen des Lösungsmittels wird mit Methylenchlorid Methanol = 98,5 : 1,5 über Kieselgel chromatographiert. Die isolierte Verbindung wird aus wenig Cyclohexan umkristallisiert. Es wurden 13 g Produkt erhalten.

a) 3-(1-Ethoxyiminobutyl)-6-(3-isopropylisoxazol-5-yl)-tetrahydro pyran-2,4-dion

3 g 3-Butyryl-6-(3-isopropylisoxazol-5-yl)-tetrahydropyran-2,4-dion wurden in 30 ml trockenem Methanol gelöst und nacheinander mit 0,9 g Natriumhydrogencarbonat und 1,1 g Ethoxyaminhydrochlorid versetzt, 12 Stunden bei Raumtemperatur gerührt und anschließend zur Trockene gebracht. Der Rückstand wird in 50 ml Methylenchlorid aufgenommen, einmal mit Wasser, zweimal mit gesättigter Natriumhydrogencarbonatlösung, nochmals mit Wasser gewaschen, mit Natriumsulfat getrocknet und das Lösungsmittel abgezogen. Es wurden 3,1 g Oximether erhalten.

Tabelle 1

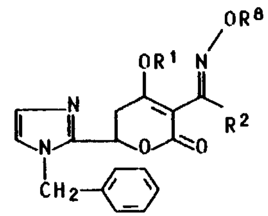

| Nr. | $R^1$ | $R^2$ | $R^8$ | physik. Daten Fp [°C]; NMR [δ, ppm] |
|---|---|---|---|---|
| 1.1 | H | Ethyl | Ethyl | |
| 1.2 | H | Ethyl | Allyl | |
| 1.3 | H | Ethyl | E-3-Chlorprop-2-enyl | |
| 1.4 | H | Ethyl | E-But-2-enyl | |
| 1.5 | H | Ethyl | 5-Chlorthien-2-ylmethyl | |
| 1.6 | H | Propyl | Ethyl | |
| 1.7 | H | Propyl | Allyl | 0.98(t,3H); 3.5(dd,1H); 4.53(dd,2H); 5.1-5.5(m,4H); 5.8-6.1(m,1H) |
| 1.8 | H | Propyl | E-3-Chlorprop-2-enyl | 0.95(t,3H); 1.55(dt,2H); 4.52(d,2H); 6.0-6.2(m,1H); 6.38(d,1H) |
| 1.9 | H | Propyl | E-But-2-enyl | 0.96(t,3H); 1.79(d,3H); 4.44(d,2H); 5.5-6.0(m,2H); 6.85-7.4(m,7H) |
| 1.10 | H | Propyl | 5-Chlorthien-2-ylmethyl | 0.94(t,3H); 1.54(dt,2H); 5.08(s,2H); 6.6-7.4(m,9H) |
| 1.11 | H | Propyl | E-4-(4-Fluorphenyl)-but-2-enyl | 0.98(t,3H); 1.58(dt,2H); 4.5(d,2H); 5.5-5.75(m,1H); 5.9-6.1(m,1H) |
| 1.12 | H | Propyl | Prop-2-inyl | 0.94(s,3H); 2.55(t,1H); 4.67(t,2H); 5.1-5.35(m,3H); 6.85-7.5(m,7H) |

Tabelle 2

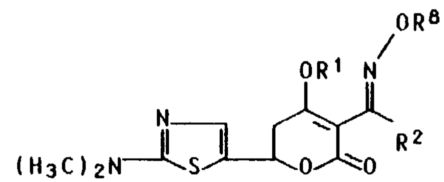

| Nr. | $R^1$ | $R^2$ | $R^8$ | physik. Daten Fp [°C]; NMR [δ, ppm] |
|---|---|---|---|---|
| 2.1 | H | Ethyl | Ethyl | |
| 2.2 | H | Ethyl | Allyl | |
| 2.3 | H | Ethyl | E-3-Chlorprop-2-enyl | |
| 2.4 | H | Ethyl | E-But-2-enyl | |
| 2.5 | H | Ethyl | 5-Chlorthien-2-ylmethyl | |
| 2.6 | H | Propyl | Ethyl | |
| 2.7 | H | Propyl | Allyl | |
| 2.8 | H | Propyl | E-3-Chlorprop-2-enyl | |
| 2.9 | H | Propyl | E-But-2-enyl | |
| 2.10 | H | Propyl | 5-Chlorthien-2-ylmethyl | |

Tabelle 3

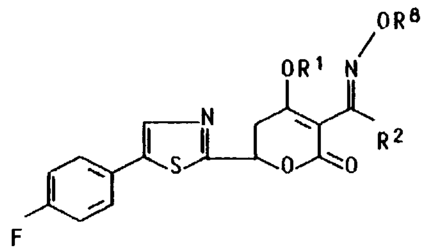

| Nr. | $R^1$ | $R^2$ | $R^8$ | physik. Daten Fp [°C]; NMR [δ, ppm] |
|---|---|---|---|---|
| 3.1 | H | Ethyl | Ethyl | |
| 3.2 | H | Ethyl | Allyl | |
| 3.3 | H | Ethyl | E-3-Chlorprop-2-enyl | |
| 3.4 | H | Ethyl | E-But-2-enyl | |
| 3.5 | H | Ethyl | 5-Chlorthien-2-ylmethyl | |
| 3.6 | H | Propyl | Ethyl | |
| 3.7 | H | Propyl | Allyl | |
| 3.8 | H | Propyl | E-3-Chlorprop-2-enyl | |
| 3.9 | H | Propyl | E-But-2-enyl | |
| 3.10 | H | Propyl | 5-Chlorthien-2-ylmethyl | |

Tabelle 4

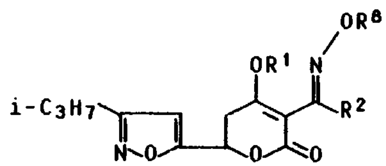

| Nr. | $R^1$ | $R^2$ | $R^8$ | physik. Daten Fp [°C]; NMR [δ,ppm] |
|---|---|---|---|---|
| 4.1 | H | Ethyl | Ethyl | 1.21(t,3H); 1.23(d,6H); 1.38(t,3H); 5.56(dd, 1H); |
| 4.2 | H | Ethyl | Allyl | 1.21(t,3H); 1.3(d,6H); 6.3(S,1H); |
| 4.3 | H | Ethyl | E-3-Chlorprop-2-enyl | 1.19(t,3H); 1.3(d,6H); 5.58(dd,1H); 6.45(d,1H); |
| 4.4 | H | Ethyl | E-But-2-enyl | 1.20(t,3H); 1.29(d,6H); 5.54(dd,1H); 6.28(S,1H); |
| 4.5 | H | Ethyl | 5-Chlorthien-2-ylmethyl | |
| 4.6 | H | Ethyl | Methyl | |
| 4.7 | H | Ethyl | Propyl | |
| 4.8 | H | Ethyl | Prop-2-inyl | |
| 4.9 | H | Ethyl | 3-Fluorpropyl | |
| 4.10 | H | Ethyl | Z-3-Chlorprop-2-enyl | |
| 4.11 | H | Ethyl | 2-Chlorprop-2-enyl | |
| 4.12 | H | Ethyl | But-2-inyl | |
| 4.13 | H | Ethyl | 2-Methoxyethyl | |
| 4.14 | H | Ethyl | E-4(4-Fluorphenyl)but-2-enyl | |
| 4.15 | H | Ethyl | E-4(4-Trifluorphenyl)but-2-enyl | |
| 4.16 | H | Ethyl | E-4(4-Chlorphenyl)but-2-enyl | |

Tabelle 4 (Fortsetzung)

| Nr. | $R^1$ | $R^2$ | $R^8$ | physik. Daten Fp [$^0C$]; NMR [$\delta$,ppm] |
|---|---|---|---|---|
| 4.17 | H | Ethyl | E-4(4-t-Butylphenyl)but-2-enyl | |
| 4.18 | H | Ethyl | E-4-Phenylbut-2-enyl- | |
| 4.19 | H | Ethyl | Benzyl | |
| 4.20 | H | Ethyl | 4-Fluorbenzyl | |
| 4.21 | H | Ethyl | 3-Trifluormethylbenzyl | |
| 4.22 | H | Ethyl | 4-Nitrobenzyl | |
| 4.23 | H | Ethyl | 4-Cyanobenzyl | |
| 4.24 | H | Ethyl | 3-Chlorbenzyl | |
| 4.25 | H | Ethyl | Thien-2-ylmethyl | |
| 4.26 | H | Ethyl | 5-Bromthien-2-ylmethyl | |
| 4.27 | H | Propyl | Ethyl | 0.99(t,3H); 1.29(d,6H); 1.35(3,tH); 5.52(dd,1H); |
| 4.28 | H | Propyl | Allyl | 0.98(t,3H); 1.27(d,6H); 4.54(d,2H); 6.27(S,1H); |
| 4.29 | H | Propyl | E-3-Chlorprop-2-enyl | 0.96(t,3H); 1.27(d,6H); 4.54(d,2H); 5.53(dd,1H); |
| 4.30 | H | Propyl | E-But-2-enyl | 0.98(t,3H); 1.28(d,6H); 1.8(d.3H); 5.52(dd,1H); |
| 4.31 | H | Propyl | 5-Chlorthien-2-ylmethyl | |
| 4.32 | H | Propyl | Methyl | |
| 4.33 | H | Propyl | Propyl | |
| 4.34 | H | Propyl | Prop-2-inyl | |
| 4.35 | H | Propyl | 3-Fluorpropyl | |
| 4.36 | H | Propyl | Z-3-Chlorprop-2-enyl | |
| 4.37 | H | Propyl | 2-Chlorprop-2-enyl | |
| 4.38 | H | Propyl | But-2-inyl | |

Tabelle 4 (Fortsetzung)

| Nr. | $R^1$ | $R^2$ | $R^8$ | physik. Daten Fp [°C]; NMR [δ, ppm] |
|---|---|---|---|---|
| 4.39 | H | Propyl | 2-Methoxyethyl | |
| 4.40 | H | Propyl | E-4(4-Fluorphenyl)but-2-enyl | |
| 4.41 | H | Propyl | E-4(4-Chlorphenyl)but-2-enyl | |
| 4.42 | H | Propyl | E-4(4-t-Butylphenyl)but-2-enyl | |
| 4.43 | H | Propyl | E-4(4-Trifluormethylphenyl-but-2-enyl | |
| 4.44 | H | Propyl | E-4-Phenylbut-2-enyl | |
| 4.45 | H | Propyl | Benzyl | |
| 4.46 | H | Propyl | 4-Fluorbenzyl | |
| 4.47 | H | Propyl | 3-Trifluormethylbenzyl | |
| 4.48 | H | Propyl | 4-Nitrobenzyl | |
| 4.49 | H | Propyl | 4-Cyanobenzyl | |
| 4.50 | H | Propyl | 3-Chlorbenzyl | |
| 4.51 | H | Propyl | Thien-2-ylmethyl | |
| 4.52 | H | Propyl | 5-Bromthien-2-ylmethyl | |

Tabelle 5

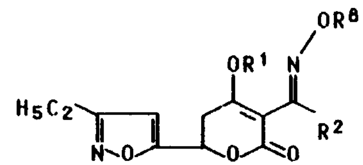

| Nr. | $R^1$ | $R^2$ | $R^8$ | physik. Daten Fp [°C]; NMR [δ, ppm] |
|---|---|---|---|---|
| 5.1 | H | Ethyl | Ethyl | |
| 5.2 | H | Ethyl | Allyl | 1.21(t,3H); 1.29(t,3H); 2.73(q,2H); 4.58(d,2H); 6.29(s,1H); |
| 5.3 | H | Ethyl | E-3-Chlorprop-2-enyl | |
| 5.4 | H | Ethyl | E-But-2-enyl | 1.19(t,3H); 1.23(t,3H); 1.81(d,3H); 4.48(d,2H); 6.28(s,1H); |
| 5.5 | H | Ethyl | 5-Chlorthien-2-ylmethyl | |
| 5.6 | H | Ethyl | Methyl | |
| 5.7 | H | Ethyl | Propyl | |
| 5.8 | H | Ethyl | Prop-2-inyl | |
| 5.9 | H | Ethyl | 3-Fluorpropyl | |
| 5.10 | H | Ethyl | Z-3-Chlorprop-2-enyl | |
| 5.11 | H | Ethyl | 2-Chlorprop-2-enyl | |
| 5.12 | H | Ethyl | But-2-inyl | |
| 5.13 | H | Ethyl | 2-Methoxyethyl | |
| 5.14 | H | Ethyl | E-4(4-t-Butylphenyl)but-2-enyl | |
| 5.15 | H | Ethyl | E-4(4-Fluorphenyl)but-2-enyl | |
| 5.16 | H | Ethyl | E-4(4-Chlorphenyl)but-2-enyl | |
| 5.17 | H | Ethyl | E-4(4-Trifluormethylphenyl)but-2-enyl | |
| 5.18 | H | Ethyl | E-4-Phenylbut-2-enyl | |

Tabelle 5 (Fortsetzung)

| Nr. | $R^1$ | $R^2$ | $R^8$ | physik. Daten Fp [°C]; NMR [δ, ppm] |
|---|---|---|---|---|
| 5.19 | H | Ethyl | Benzyl | |
| 5.20 | H | Ethyl | 4-Fluorbenzyl | |
| 5.21 | H | Ethyl | 3-Trifluormethylbenzyl | |
| 5.22 | H | Ethyl | 4-Nitrobenzyl | |
| 5.23 | H | Ethyl | 4-Cyanobenzyl | |
| 5.24 | H | Ethyl | 3-Chlorbenzyl | |
| 5.25 | H | Ethyl | Thien-2-ylmethyl | |
| 5.26 | H | Ethyl | 5-Bromthien-2-ylmethyl | |
| 5.27 | H | Propyl | Ethyl | |
| 5.28 | H | Propyl | Allyl | |
| 5.29 | H | Propyl | E-3-Chlorprop-2-enyl | |
| 5.30 | H | Propyl | E-But-2-enyl | |
| 5.31 | H | Propyl | 5-Chlorthien-2-ylmethyl | |
| 5.32 | H | Propyl | Methyl | |
| 5.33 | H | Propyl | Propyl | |
| 5.34 | H | Propyl | Prop-2-inyl | |
| 5.35 | H | Propyl | 3-Fluorpropyl | |
| 5.36 | H | Propyl | Z-3-Chlorprop-2-enyl | |
| 5.37 | H | Propyl | 2-Chlorprop-2-enyl | |
| 5.38 | H | Propyl | But-2-inyl | |
| 5.39 | H | Propyl | 2-Methoxyethyl | |
| 5.40 | H | Propyl | E-4(4-t-Butylphenyl)but-2-enyl | |
| 5.41 | H | Propyl | E-4(4-Fluorphenyl)but-2-enyl | |
| 5.42 | H | Propyl | E-4(4-Chlorphenyl)but-2-enyl | |
| 5.43 | H | Propyl | E-4(4-Trifluormethylphenyl)but-2-enyl | |
| 5.44 | H | Propyl | E-4-Phenylbut-2-enyl | |

Tabelle 5 (Fortsetzung)

| Nr. | $R^1$ | $R^2$ | $R^8$ | physik. Daten Fp [°C]; NMR [δ, ppm] |
|---|---|---|---|---|
| 5.45 | H | Propyl | Benzyl | |
| 5.46 | H | Propyl | 4-Fluorbenzyl | |
| 5.47 | H | Propyl | 3-Trifluormethylbenzyl | |
| 5.48 | H | Propyl | 4-Nitrobenzyl | |
| 5.49 | H | Propyl | 4-Cyanobenzyl | |
| 5.50 | H | Propyl | 3-Chlorbenzyl | |
| 5.51 | H | Propyl | Thien-2-ylmethyl | |
| 5.52 | H | Propyl | 5-Bromthien-2-ylmethyl | |

Tabelle 6

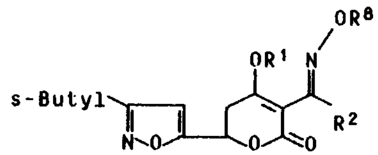

| Nr. | $R^1$ | $R^2$ | $R^8$ | physik. Daten Fp [°C]; NMR [δ, ppm] |
|---|---|---|---|---|
| 6.1 | H | Ethyl | Ethyl | 0.88(t,3H); 1.36(t,3H); 4.12(q,2H); 5.53(dd,1H); 6.23(s,1H) |
| 6.2 | H | Ethyl | Allyl | 0.89(t,3H); 1.05(m,6H); 4.55(d,2H); 5.37-5.6(m,3H); 6.26(s,1H) |
| 6.3 | H | Ethyl | E-3-Chlorprop-2-enyl | 0.87(t,3H); 1.16(t,3H); 1.25(d,3H); 4.55(d,2H); 5.54(dd,1H); 6.27(s,1H) |
| 6.4 | H | Ethyl | E-But-2-enyl | 0.86(t,3H); 1.17(t,3H); 1.26(d,3H); 5.52(dd,1H); 6.26(s,1H) |
| 6.5 | H | Ethyl | 5-Chlorthien-2-ylmethyl | 0.88(t,3H); 1.12(t,3H); 1.25(d,3H); 5.09(s,2H); 5.5(dd,1H) |
| 6.6 | H | Ethyl | Methyl | |
| 6.7 | H | Ethyl | Propyl | |
| 6.8 | H | Ethyl | Prop-2-inyl | |
| 6.9 | H | Ethyl | 3-Fluorpropyl | |
| 6.10 | H | Ethyl | Z-3-Chlorprop-2-enyl | |
| 6.11 | H | Ethyl | 2-Chlorprop-2-enyl | |
| 6.12 | H | Ethyl | But-2-inyl | |
| 6.13 | H | Ethyl | E-4(4-t-Butylphenyl)but-2-enyl | |
| 6.14 | H | Ethyl | 2-Methoxyethyl | |
| 6.15 | H | Ethyl | E-4(4-Fluorphenyl)but-2-enyl | |
| 6.16 | H | Ethyl | E-4(4-Chlorphenyl)but-2-enyl | |

Tabelle 6 (Fortsetzung)

| Nr. | $R^1$ | $R^2$ | $R^8$ | physik. Daten Fp [°C]; NMR [δ,ppm] |
|---|---|---|---|---|
| 6.17 | H | Ethyl | E-4(4-Trifluormethylphenyl)but-2-enyl | |
| 6.18 | H | Ethyl | E-4-Phenylbut-2-enyl | |
| 6.19 | H | Ethyl | Benzyl | |
| 6.20 | H | Ethyl | 4-Fluorbenzyl | |
| 6.21 | H | Ethyl | 3-Trifluormethylbenzyl | |
| 6.22 | H | Ethyl | 4-Nitrobenzyl | |
| 6.23 | H | Ethyl | 4-Cyanobenzyl | |
| 6.24 | H | Ethyl | 3-Chlorbenzyl | |
| 6.25 | H | Ethyl | Thien-2-ylmethyl | |
| 6.26 | H | Ethyl | 5-Bromthien-2-ylmethyl | |
| 6.27 | H | Propyl | Ethyl | 0.9(t,3H); 1.0(t,3H); 1.25(d,3H); 1.37(t,3H); 4.13(q,2H); 5.54(dd,1H) |
| 6.28 | H | Propyl | Allyl | 0.86(t,3H); 0.95(t,3H); 1.27(d,3H); 4.54(d,2H); 5.3-5.6(m,3H); 6.28(s,1H) |
| 6.29 | H | Propyl | E-3-Chlorprop-2-enyl | 0.89(t,3H); 1.0(t,3H); 1.25(d,3H); 1.8(d,3H); 4.46(d,2H); 5.5(dd,1H) |
| 6.30 | H | Propyl | E-But-2-enyl | 0.9(t,3H); 0.99(t,3H); 1.26(d,3H); 4.54(d,2H); 5.54(dd,1H) |
| 6.31 | H | Propyl | 5-Chlorthien-2-ylmethyl | 0.9(t,3H); 0.99(t,3H); 1.25(d,3H); 5.1(s,2H); 5.5(dd,1H); 6.24(s,1H) |
| 6.32 | H | Propyl | Methyl | |
| 6.33 | H | Propyl | Propyl | |
| 6.34 | H | Propyl | Prop-2-inyl | 0.86(t,3H); 0.95(t,3H); 1.26(d,3H); 2.59(t,1H); 4.67(d,2H); 5.53(dd,1H) |
| 6.35 | H | Propyl | 3-Fluorpropyl | |
| 6.36 | H | Propyl | Z-3-Chlorprop-2-enyl | |

Tabelle 6 (Fortsetzung)

| Nr. | $R^1$ | $R^2$ | $R^8$ | physik. Daten Fp [°C]; NMR [δ,ppm] |
|---|---|---|---|---|
| 6.37 | H | Propyl | 2-Chlorprop-2-enyl | |
| 6.38 | H | Propyl | But-2-inyl | |
| 6.39 | H | Propyl | 2-Methoxyethyl | |
| 6.40 | H | Propyl | E-4(4-t-Butylphenyl)but-2-enyl | 0.89(t,3H); 0.99(t,3H); 1.26(d,3H); 3.47(d,2H); 4.52(d,2H); 5.5(dd,1H) |
| 6.41 | H | Propyl | E-4(4-Fluorphenyl)but-2-enyl | 0.86(t,3H); 0.97(t,3H); 1.27(d,3H); 3.45(d,2H); 4.5(d,2H); 5.53(dd,1H) |
| 6.42 | H | Propyl | E-4(4-Chlorphenyl)but-2-enyl | |
| 6.43 | H | Propyl | E-4(4-Trifluormethylphenyl)but-2-enyl | |
| 6.44 | H | Propyl | E-4-Phenylbut-2-enyl | |
| 6.45 | H | Propyl | Benzyl | |
| 6.46 | H | Propyl | 4-Fluorbenzyl | |
| 6.47 | H | Propyl | 3-Trifluormethylbenzyl | |
| 6.48 | H | Propyl | 4-Nitrobenzyl | |
| 6.49 | H | Propyl | 4-Cyanobenzyl | |
| 6.50 | H | Propyl | 3-Chlorbenzyl | |
| 6.51 | H | Propyl | Thien-2-ylmethyl | |
| 6.52 | H | Propyl | 5-Bromthien-2-ylmethyl | |
| 6.53 | H | Propyl | 4-Chlorbenzyl | 0.88(t,3H); 0.94(t,3H); 1.25(d,3H); 5.0(s,2H); 5.5(dd,1H); 6.23(s,1H); |

Tabelle 7

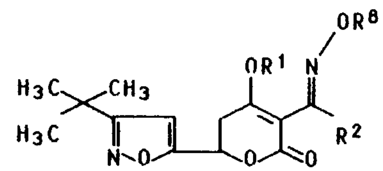

| Nr. | $R^1$ | $R^2$ | $R^8$ | physik. Daten Fp [°C]; NMR [δ,ppm] |
|---|---|---|---|---|
| 7.1 | H | Ethyl | Ethyl | |
| 7.2 | H | Ethyl | Allyl | |
| 7.3 | H | Ethyl | E-3-Chlorprop-2-enyl | |
| 7.4 | H | Ethyl | E-But-2-enyl | |
| 7.5 | H | Ethyl | 5-Chlorthien-2-ylmethyl | |
| 7.6 | H | Ethyl | Methyl | |
| 7.7 | H | Ethyl | Propyl | |
| 7.8 | H | Ethyl | Prop-2-inyl | |
| 7.9 | H | Ethyl | 3-Fluorpropyl | |
| 7.10 | H | Ethyl | Z-3-Chlorprop-2-enyl | |
| 7.11 | H | Ethyl | 2-Chlorprop-2-enyl | |
| 7.12 | H | Ethyl | But-2-inyl | |
| 7.13 | H | Ethyl | 2-Methoxyethyl | |
| 7.14 | H | Ethyl | E-4(4-t-Butylphenyl)but-2-enyl | |
| 7.15 | H | Ethyl | E-4(4-Fluorphenyl)but-2-enyl | |
| 7.16 | H | Ethyl | E-4(4-Chlorphenyl)but-2-enyl | |
| 7.17 | H | Ethyl | E-4(4-Trifluormethylphenyl)but-2-enyl | |
| 7.18 | H | Ethyl | E-4-Phenylbut-2-enyl | |
| 7.19 | H | Ethyl | Benzyl | |
| 7.20 | H | Ethyl | 4-Fluorbenzyl | |

Tabelle 7 (Fortsetzung)

| Nr. | $R^1$ | $R^2$ | $R^8$ | physik. Daten Fp [°C]; NMR [δ, ppm] |
|---|---|---|---|---|
| 7.21 | H | Ethyl | 3-Trifluormethylbenzyl | |
| 7.22 | H | Ethyl | 4-Nitrobenzyl | |
| 7.23 | H | Ethyl | 4-Cyanobenzyl | |
| 7.24 | H | Ethyl | 3-Chlorbenzyl | |
| 7.25 | H | Ethyl | Thien-2-ylmethyl | |
| 7.26 | H | Ethyl | 5-Bromthien-2-ylmethyl | |
| 7.27 | H | Propyl | Ethyl | |
| 7.28 | H | Propyl | Allyl | |
| 7.29 | H | Propyl | E-3-Chlorprop-2-enyl | |
| 7.30 | H | Propyl | E-But-2-enyl | |
| 7.31 | H | Propyl | 5-Chlorthien-2-ylmethyl | |
| 7.32 | H | Propyl | Methyl | |
| 7.33 | H | Propyl | Propyl | |
| 7.34 | H | Propyl | Prop-2-inyl | |
| 7.35 | H | Propyl | 3-Fluorpropyl | |
| 7.36 | H | Propyl | Z-3-Chlorprop-2-enyl | |
| 7.37 | H | Propyl | 2-Chlorprop-2-enyl | |
| 7.38 | H | Propyl | But-2-inyl | |
| 7.39 | H | Propyl | 2-Methoxyethyl | |
| 7.40 | H | Propyl | E-4(4-t-Butylphenyl)but-2-enyl | |
| 7.41 | H | Propyl | E-4(4-Fluorphenyl)but-2-enyl | |
| 7.42 | H | Propyl | E-4(4-Chlorphenyl)but-2-enyl | |
| 7.43 | H | Propyl | E-4(4-Trifluormethylphenyl)but-2-enyl | |
| 7.44 | H | Propyl | E-4-Phenylbut-2-enyl | |
| 7.45 | H | Propyl | Benzyl | |
| 7.46 | H | Propyl | 4-Fluorbenzyl | |

Tabelle 7 (Fortsetzung)

| Nr. | $R^1$ | $R^2$ | $R^8$ | physik. Daten Fp [°C]; NMR [δ,ppm] |
|---|---|---|---|---|
| 7.47 | H | Propyl | 3-Trifluormethylbenzyl | |
| 7.48 | H | Propyl | 4-Nitrobenzyl | |
| 7.49 | H | Propyl | 4-Cyanobenzyl | |
| 7.50 | H | Propyl | 3-Chlorbenzyl | |
| 7.51 | H | Propyl | Thien-2-ylmethyl | |
| 7.52 | H | Propyl | 5-Bromthien-2-ylmethyl | |

Tabelle 8

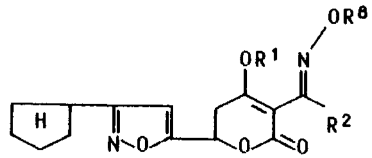

| Nr. | $R^1$ | $R^2$ | $R^8$ | physik. Daten Fp [°C]; NMR [δ,ppm] |
|---|---|---|---|---|
| 8.1 | H | Ethyl | Ethyl | |
| 8.2 | H | Ethyl | Allyl | 1.19(t,3H); 1.5-2.2(m,8H); 4.55(d,2H); 6.24(s,1H) |
| 8.3 | H | Ethyl | E-3-Chlorprop-2-enyl | 1.12(t,3H); 1.5-2.2(m,8H); 4.55(d,2H); 5.54(dd,1H) 6.26(s,1H) |
| 8.4 | H | Ethyl | E-But-2-enyl | |
| 8.5 | H | Ethyl | 5-Chlorthien-2-ylmethyl | |
| 8.6 | H | Ethyl | Methyl | |
| 8.7 | H | Ethyl | Propyl | |
| 8.8 | H | Ethyl | Prop-2-inyl | |
| 8.9 | H | Ethyl | 3-Fluorpropyl | |
| 8.10 | H | Ethyl | Z-3-Chlorprop-2-enyl | |
| 8.11 | H | Ethyl | 2-Chlorprop-2-enyl | |
| 8.12 | H | Ethyl | But-2-inyl | |
| 8.13 | H | Ethyl | 2-Methoxyethyl | |
| 8.14 | H | Ethyl | E-4(4-t-Butylphenyl)but-2-enyl | |
| 8.15 | H | Ethyl | E-4(4-Fluorphenyl)but-2-enyl | |
| 8.16 | H | Ethyl | E-4(4-Chlorphenyl)but-2-enyl | |
| 8.17 | H | Ethyl | E-4(4-Trifluormethylphenyl)but-2-enyl | |
| 8.18 | H | Ethyl | E-4-Phenylbut-2-enyl | |
| 8.19 | H | Ethyl | Benzyl | |
| 8.20 | H | Ethyl | 4-Fluorbenzyl | |
| 8.21 | H | Ethyl | 3-Trifluormethylbenzyl | |

Tabelle 8 (Fortsetzung)

| Nr. | $R^1$ | $R^2$ | $R^8$ | physik. Daten Fp [°C]; NMR [δ, ppm] |
|---|---|---|---|---|
| 8.22 | H | Ethyl | 4-Nitrobenzyl | |
| 8.23 | H | Ethyl | 4-Cyanobenzyl | |
| 8.24 | H | Ethyl | 3-Chlorbenzyl | |
| 8.25 | H | Ethyl | Thien-2-ylmethyl | |
| 8.26 | H | Ethyl | 5-Bromthien-2-ylmethyl | |
| 8.27 | H | Propyl | Ethyl | |
| 8.28 | H | Propyl | Allyl | |
| 8.29 | H | Propyl | E-3-Chlorprop-2-enyl | |
| 8.30 | H | Propyl | E-But-2-enyl | |
| 8.31 | H | Propyl | 5-Chlorthien-2-ylmethyl | |
| 8.32 | H | Propyl | Methyl | |
| 8.33 | H | Propyl | Propyl | |
| 8.34 | H | Propyl | Prop-2-inyl | |
| 8.35 | H | Propyl | 3-Fluorpropyl | |
| 8.36 | H | Propyl | Z-3-Chlorprop-2-enyl | |
| 8.37 | H | Propyl | 2-Chlorprop-2-enyl | |
| 8.38 | H | Propyl | But-2-inyl | |
| 8.39 | H | Propyl | 2-Methoxyethyl | |
| 8.40 | H | Propyl | E-4(4-Butylphenyl)but-2-enyl | |
| 8.41 | H | Propyl | E-4(4-Fluorphenyl)but-2-enyl | |
| 8.42 | H | Propyl | E-4(4-Chlorphenyl)but-2-enyl | |
| 8.43 | H | Propyl | E-4(4-Trifluormethylphenyl)but-2-enyl | |
| 8.44 | H | Propyl | E-4-Phenylbut-2-enyl | |
| 8.45 | H | Propyl | Benzyl | |
| 8.46 | H | Propyl | 4-Fluorbenzyl | |
| 8.47 | H | Propyl | 3-Trifluormethylbenzyl | |

Tabelle 8 (Fortsetzung)

| Nr. | $R^1$ | $R^2$ | $R^8$ | physik. Daten Fp [°C]; NMR [δ,ppm] |
|---|---|---|---|---|
| 8.48 | H | Propyl | 4-Nitrobenzyl | |
| 8.49 | H | Propyl | 4-Cyanobenzyl | |
| 8.50 | H | Propyl | 3-Chlorbenzyl | |
| 8.51 | H | Propyl | Thien-2-ylmethyl | |
| 8.52 | H | Propyl | 5-Bromthien-2-ylmethyl | |

Tabelle 9

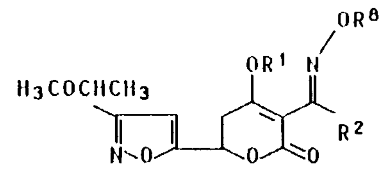

| Nr. | $R^1$ | $R^2$ | $R^8$ | physik. Daten Fp [°C]; NMR [δ, ppm] |
|---|---|---|---|---|
| 9.1 | H | Ethyl | Ethyl | |
| 9.2 | H | Ethyl | Allyl | |
| 9.3 | H | Ethyl | E-3-Chlorprop-2-enyl | |
| 9.4 | H | Ethyl | E-But-2-enyl | |
| 9.5 | H | Ethyl | 5-Chlorthien-2-ylmethyl | |
| 9.6 | H | Ethyl | Methyl | |
| 9.7 | H | Ethyl | Propyl | |
| 9.8 | H | Ethyl | Prop-2-inyl | |
| 9.9 | H | Ethyl | 3-Fluorpropyl | |
| 9.10 | H | Ethyl | Z-3-Chlorprop-2-enyl | |
| 9.11 | H | Ethyl | 2-Chlorprop-2-enyl | |
| 9.12 | H | Ethyl | But-2-inyl | |
| 9.13 | H | Ethyl | 2-Methoxyethyl | |
| 9.14 | H | Ethyl | E-4(4-Butylphenyl)but-2-enyl | |
| 9.15 | H | Ethyl | E-4(4-Fluorphenyl)but-2-enyl | |
| 9.16 | H | Ethyl | E-4(4-Chlorphenyl)but-2-enyl | |
| 9.17 | H | Ethyl | E-4(4-Trifluormethylphenyl)but-2-enyl | |
| 9.18 | H | Ethyl | E-4-Phenylbut-2-enyl | |
| 9.19 | H | Ethyl | Benzyl | |
| 9.20 | H | Ethyl | 4-Fluorbenzyl | |

Tabelle 9 (Fortsetzung)

| Nr. | $R^1$ | $R^2$ | $R^8$ | physik. Daten Fp [°C]; NMR [δ, ppm] |
|---|---|---|---|---|
| 9.21 | H | Ethyl | 3-Trifluormethylbenzyl | |
| 9.22 | H | Ethyl | 4-Nitrobenzyl | |
| 9.23 | H | Ethyl | 4-Cyanobenzyl | |
| 9.24 | H | Ethyl | 3-Chlorbenzyl | |
| 9.25 | H | Ethyl | Thien-2-ylmethyl | |
| 9.26 | H | Ethyl | 5-Bromthien-2-ylmethyl | |
| 9.27 | H | Propyl | Ethyl | |
| 9.28 | H | Propyl | Allyl | |
| 9.29 | H | Propyl | E-3-Chlorprop-2-enyl | |
| 9.30 | H | Propyl | E-But-2-enyl | |
| 9.31 | H | Propyl | 5-Chlorthien-2-ylmethyl | |
| 9.32 | H | Propyl | Methyl | |
| 9.33 | H | Propyl | Propyl | |
| 9.34 | H | Propyl | Prop-2-inyl | |
| 9.35 | H | Propyl | 3-Fluorpropyl | |
| 9.36 | H | Propyl | Z-3-Chlorprop-2-enyl | |
| 9.37 | H | Propyl | 2-Chlorprop-2-enyl | |
| 9.38 | H | Propyl | But-2-inyl | |
| 9.39 | H | Propyl | 2-Methoxyethyl | |
| 9.40 | H | Propyl | E-4(4-t-Butylphenyl)but-2-enyl | |
| 9.41 | H | Propyl | E-4(4-Fluorphenyl)but-2-enyl | |
| 9.42 | H | Propyl | E-4(4-Chlorphenyl)but-2-enyl | |
| 9.43 | H | Propyl | E-4(4-Trifluormethylphenyl)but-2-enyl | |
| 9.44 | H | Propyl | E-4-Phenylbut-2-enyl | |
| 9.45 | H | Propyl | Benzyl | |
| 9.46 | H | Propyl | 4-Fluorbenzyl | |

Tabelle 9 (Fortsetzung)

| Nr. | $R^1$ | $R^2$ | $R^8$ | physik. Daten Fp [°C]; NMR [δ,ppm] |
|---|---|---|---|---|
| 9.47 | H | Propyl | 3-Trifluormethylbenzyl | |
| 9.48 | H | Propyl | 4-Nitrobenzyl | |
| 9.49 | H | Propyl | 4-Cyanobenzyl | |
| 9.50 | H | Propyl | 3-Chlorbenzyl | |
| 9.51 | H | Propyl | Thien-2-ylmethyl | |
| 9.52 | H | Propyl | 5-Bromthien-2-ylmethyl | |

Tabelle 10

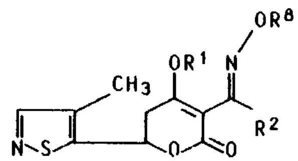

| Nr. | $R^1$ | $R^2$ | $R^8$ | physik. Daten Fp [°C]; NMR [δ,ppm] |
|---|---|---|---|---|
| 10.1 | H | Ethyl | Ethyl | |
| 10.2 | H | Ethyl | Allyl | |
| 10.3 | H | Ethyl | E-3-Chlorprop-2-enyl | |
| 10.4 | H | Ethyl | E-But-2-enyl | |
| 10.5 | H | Ethyl | 5-Chlorthien-2-ylmethyl | |
| 10.6 | H | Propyl | Ethyl | 1.02(t,3H); 1.39(t,3H); 2.3(s,3H); 4.14(q,2H); 5.47(dd,1H) |
| 10.7 | H | Propyl | Allyl | 1.02(t,3H); 2.3(s,3H); 4.56(d,2H); 5.25-5.55(m,2H); 5.7(dd,1H) |
| 10.8 | H | Propyl | E-3-Chlorprop-2-enyl | 1.0(t,3H); 2.28(s,3H); 4.55(d,2H); 5.71(dd,1H); 6.0-6.2(m,1H) |
| 10.9 | H | Propyl | E-But-2-enyl | 1.03(t,3H); 1.82(d,3H); 2.3(s,3H); 4.47(d,2H); 5.55-5.75(m,2H) |
| 10.10 | H | Propyl | 5-Chlorthien-2-ylmethyl | |

Tabelle 11

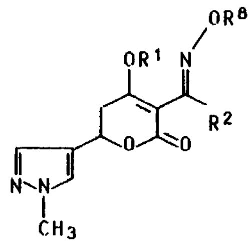

| Nr. | $R^1$ | $R^2$ | $R^8$ | physik. Daten Fp [°C]; NMR [δ, ppm] |
|---|---|---|---|---|
| 11.1 | H | Ethyl | Ethyl | |
| 11.2 | H | Ethyl | Allyl | 1.2(t,3H); 3.9(s,3H); 4.55(d,2H); 5.2-5.6(m,3H); 5.85-6.15(m,1H) |
| 11.3 | H | Ethyl | E-3-Chlorprop-2-enyl | 1.16(t,3H); 3.9(s,3H); 4.55(d,2H); 5.42(dd,1H); 6.0-6.2(m,1H) |
| 11.4 | H | Ethyl | E-But-2-enyl | 1.2(t,3H); 1.78(d,3H); 3.9(s,3H); 4.47(d,2H); 5.4(dd,1H) |
| 11.5 | H | Ethyl | 5-Chlorthien-2-ylmethyl | 1.16(t,3H); 3.9(s,3H); 5.1(s,2H); 5.4(dd,1H); 6.83(d,1H) |
| 11.6 | H | Propyl | Ethyl | |
| 11.7 | H | Propyl | Allyl | |
| 11.8 | H | Propyl | E-3-Chlorprop-2-enyl | |
| 11.9 | H | Propyl | E-But-2-enyl | |
| 11.10 | H | Propyl | 5-Chlorthien-2-ylmethyl | |
| 11.11 | H | Ethyl | E-4-(4-Fluorphenyl)-but-2-enyl | 1.19(t,3H); 3.42(d,2H); 3.88(s,3H); 4.5(d,2H); 5.4(dd,1H) |
| 11.12 | H | Ethyl | 3-Trifluormethylphenyl | 1.18(t,3H); 3.9(s,3H); 5.14(s,2H); 5.4(dd,1H); 7.4-7.7(m,6H) |
| 11.13 | H | Ethyl | But-2-inyl | 1.19(t,3H); 1.9(t,3H); 3.91(s,3H); 4.62(d,2H); 5.44(dd,1H) |
| 11.14 | H | Ethyl | Prop-2-inyl | 0.86(t,3H); 0.95(t,3H); 1.26(d,3H); 2.59(t,1H); 4.67(d,2H); 5.53(dd,1H) |

## Anwendungsbeispiele

Die herbizide Wirkung der Tetrahydropyran-2,4-dionderivate der Formel I, in denen Z eine Oximether-Gruppe (-$NOR^8$) bedeutet, ließ sich durch Gewächshausversuche zeigen:

Zur Aufzucht der Testpflanzen dienten Plastikblumentöpfe mit 300 $cm^3$ Inhalt und lehmigem Sand mit etwa 3 % Humus als Substrat; zur Anzucht von Sojapflanzen wurde Torf zugesetzt. Die Samen der Testpflanzen wurden nach Arten getrennt flach eingesät.

Bei Vorauflaufbehandlung wurden die aufbereiteten Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach wurden die Gefäße mit durchsichtigen Plastikhauben abgedeckt, bis die Pflanzen angewachsen waren. Diese Abdeckung fördert ein gleichmäßiges Keimen der Testpflanzen, sofern dies nicht durch die Wirkstoffe beeinträchtigt wird.

Zum Zwecke der Nachauflaufbehandlung wurden die Testpflanzen je nach Wuchsform erst bis zu einer Wuchshöhe von 3 bis 15 cm angezüchtet und erst dann mit den in Wasser suspendierten oder emulgierten Wirkstoffen behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmenge für die Nachauflaufbehandlung betrug 0,5 und 0,25 kg Wirkstoff/ha. Eine Abdeckung unterblieb bei der Nachauflaufbehandlung.

Die Versuchsgefäße wurden im Gewächshaus aufgestellt, wobei für wärmeliebende Arten wärmere Bereiche (20 bis 35°C) und für solche gemäßigter Klimate 10 bis 25°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürzung | Lateinischer Name | Deutscher Name | Englischer Name |
|---|---|---|---|
| ALOMY | Alopecuzus myosuroides | Ackerfuchsschwanz | blackgrass |
| AVEFA | Avena fatua | Flughafer | wild oats |
| ECHCG | Echinochloa crus-galli | Hühnerhurse | bernyardgrass |
| SETFA | Setaria fatua | Flughafer | wild oats |
| SETVI | Setaria viridis | Grüne Borstenhirse | green foxtail |
| TRZAW | Triticum aestivum | Weizen | wheat |

Der beispielhaft ausgewählte Wirkstoff 4.1 zeigt bei Anwendung von 0,5 kg a.S./ha im Nachauflaufverfahren herbizide Aktivität gegen grasartige Pflanzen bei gleichzeitiger Verträglichkeit für eine Kulturpflanze. Verbindung 4.3 bekämpft bei Aufwandmengen von 0,25 kg/ha a.S. im Gewächshaus unerwünschte Gräser.

Die Verbindungen I, in denen Z eine Oximether-Gruppe ($-NOR^8$) bedeutet, sind verträglich und somit selektiv in breitblättrigen Kulturen sowie in monokotylen Gewächsen, welche nicht zu den Gramineen zählen. Außerdem eignen sie sich zur Bekämpfung unerwünschter Gräser in Gramineenkulturen wie Weizen und Reis.

## Patentansprüche

**1.** Tetrahydropyran-2,4-dione der Formel I

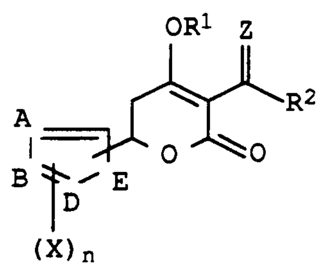

I

in der die Substituenten und der Index folgende Bedeutung haben

$R^1$

Wasserstoff;

$R^2$
$C_1$-$C_6$-Alkyl;
A, B, D
CH oder N, wobei mindestens eine der Variablen für Stickstoff steht,
E
Sauerstoff, Schwefel oder -$NR^7$-, wobei
$R^7$ für
$C_1$-$C_6$-Alkyl; Benzyl oder Phenyl, welches ein- bis dreifach durch Halogen und/oder $C_1$-$C_6$-Halogenalkyl substituiert sein kann,
steht;
X
Halogen, $C_1$-$C_6$-Alkyl oder $C_3$-$C_6$-Cycloalkyl, welche ein- bis dreifach durch Halogen, $C_1$-$C_6$-Alkoxy und/oder $C_1$-$C_6$-Alkylthio substituiert sein können; $C_2$-$C_6$-Alkenyl; $C_2$-$C_6$-Alkinyl; $C_1$-$C_6$-Alkoxy; $C_2$-$C_6$-Alkenyloxy; $C_1$-$C_6$-Alkylthio; $C_1$-$C_6$-Alkoxycarbonyl; $C_2$-$C_6$-Alkanoyloxy; $NR^5R^6$; Phenyl, welches ein- bis dreifach durch Halogen, Nitro, Cyano, $C_1$-$C_6$-Alkyl, $C_1$-$C_6$-Alkoxy, $C_1$-$C_6$-Halogenalkyl und/oder $NR^3R^4$ substituiert sein kann:
n
0, 1 oder 2, wobei die Reste X im Falle von n = 2 auch verschieden sein können;
$R^3$, $R^4$, $R^5$, $R^6$
Wasserstoff, $C_1$-$C_6$-Alkyl;
Z
Sauerstoff oder ein Oximether-Rest -$NOR^8$, wobei
$R^8$ für
$C_1$-$C_6$-Alkyl oder $C_2$-$C_6$-Alkenyl, welche ein- bis dreifach durch Halogen, $C_1$-$C_6$-Alkoxy und/oder $C_1$-$C_6$-Alkylthio substituiert sein können; $C_2$-$C_6$-Alkinyl; $C_2$-$C_6$-Halogenalkinyl; $C_1$-$C_6$-Alkyl oder $C_2$-$C_6$-Alkenyl, jeweils substituiert bis dreifach durch Phenyl, welches ein- bis dreifach durch Halogen, Nitro, Cyano, $C_1$-$C_6$-Alkyl, $C_1$-$C_6$-Halogenalkyl, $C_1$-$C_6$-Alkoxy und/oder $C_1$-$C_6$-Alkylthio substituiert sein kann; Thenyl, welches ein- bis dreifach durch Halogen substituiert sein kann,
steht,
sowie deren umweltverträglichen Salze.

2. Tetrahydropyrandione der Formel Ia,

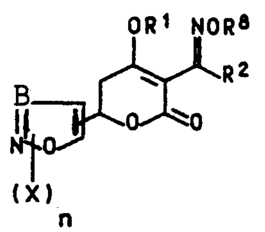

Ia

wobei B, $R^1$, $R^2$, $R^8$, X und n die in Anspruch 1 angegebene Bedeutung haben.

3. Herbizides Mittel, enthaltend ein Tetrahydropyran-2,4-dion der Formel I gemäß Anspruch 1, in der Z den Rest -$NOR^8$ bedeutet.

4. Pflanzenwachstumsregulierendes Mittel, enthaltend ein Tetrahydropyran-2,4-dion der Formel I gemäß Anspruch 1, in der Z Sauerstoff bedeutet.

5. Verfahren zur Bekämpfung von unerwünschtem Pflanzenwuchs, dadurch gekennzeichnet, daß man die unerwünschten Pflanzen und/oder die von unerwünschtem Pflanzenwachstum freizuhaltende Fläche mit einer herbizid wirksamen Menge eines Tetrahydropyran-2,4-dions der Formel I gemäß Anspruch 1, in der Z den Rest -$NOR^8$ bedeutet, behandelt.

6. Verfahren zur Regulierung des Pflanzenwachstums, dadurch gekennzeichnet, daß man eine regulativ wirksame Menge eines Tetrahydropyran-2,4-dions der Formel I gemäß Anspruch 1, in der Z Sauerstoff bedeutet, auf die Samen, die Pflanzen und/oder deren Lebensraum einwirken läßt.

7. 3-[1-(Cyclopent-2-enyloxyimino)propyl]-6-[1-(3-trifluormethylphenyl)pyrrol-3-yl]-tetrahydropyran-2,4-dion.

8. Tetrahydropyran-2,4-dione nach Anspruch 1, wobei $R^1$, $R^2$, $R^7$, X, n und Z die in Anspruch 1 angegebene Bedeutung haben, und wobei der Tetrahydropyran-2,4-dion-Grundkörper zusammen mit dem Heterocyclus in 6-Position eine der Strukturen I.1 bis I.33 bildet:

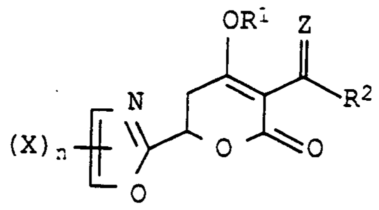

I.1

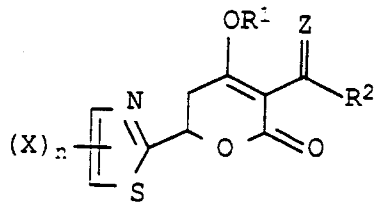

I.2

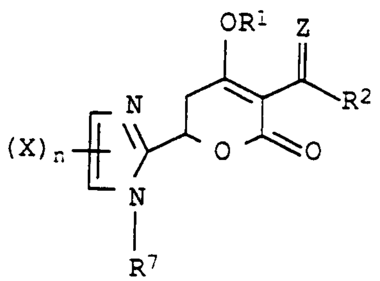

I.3

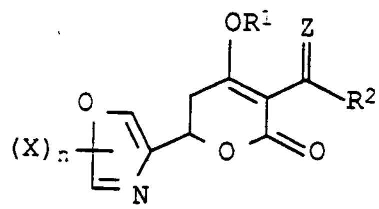

I.4

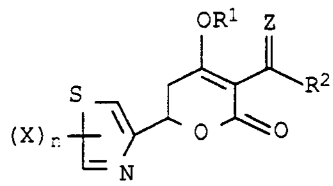

I.5

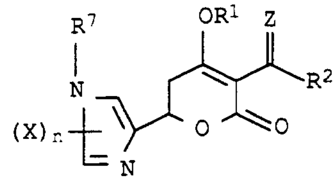

I.6

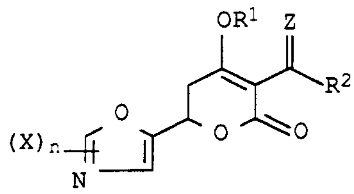

I.7

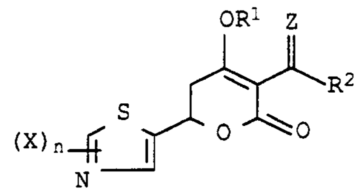

I.8

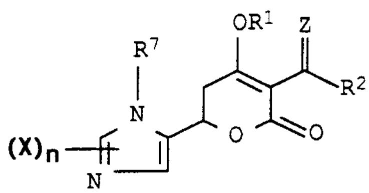

I.9

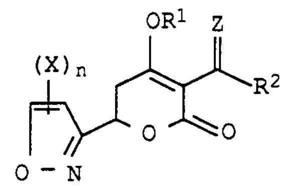

I.10

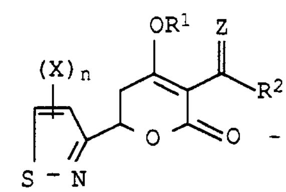

I.11

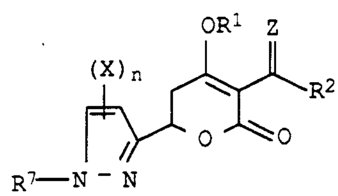

I.12

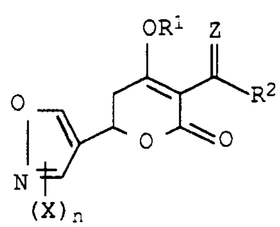

I.13

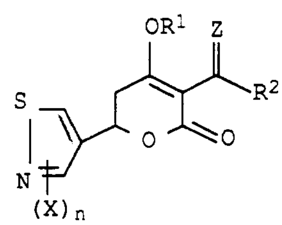

I.14

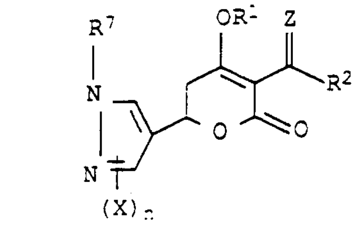

I.15

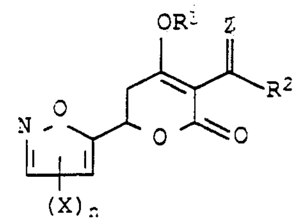

I.16

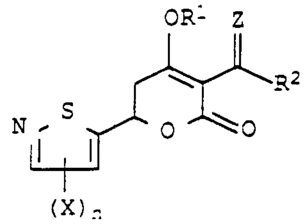

I.17

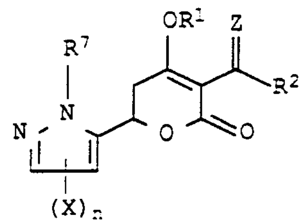

I.18

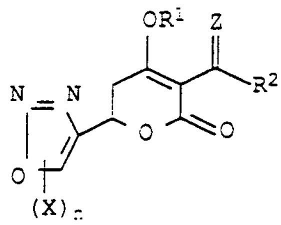

I.19

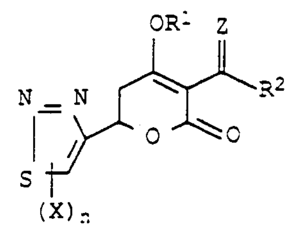

I.20

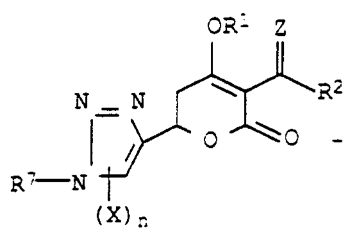

I.21

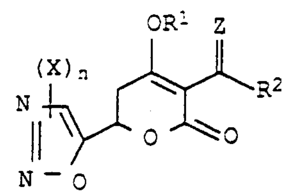

I.22

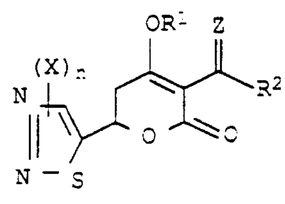

I.23

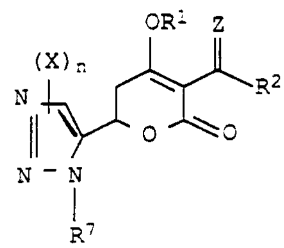

I.24

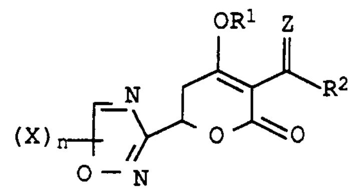

I.25

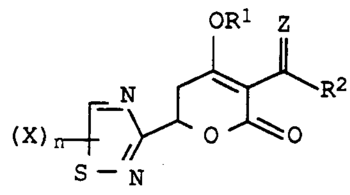

I.26

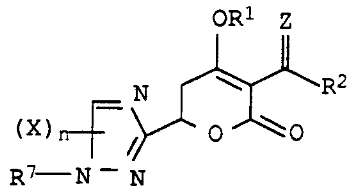

I.27

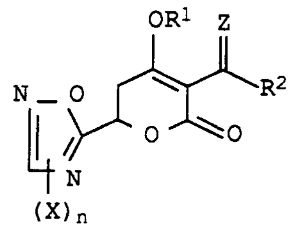

I.28

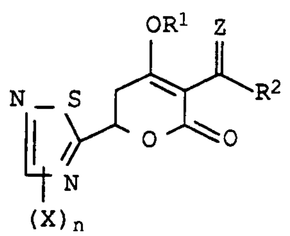

I.29

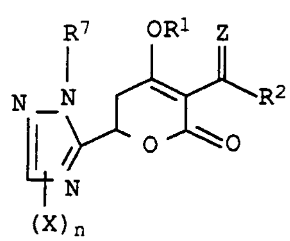

I.30

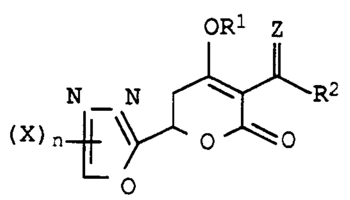

I.31

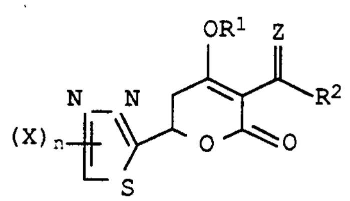

I.32

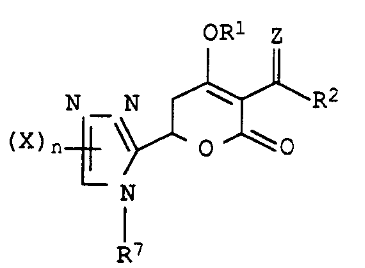

I.33

## Claims

**1.** Tetrahydropyran-2,4-diones of the formula I

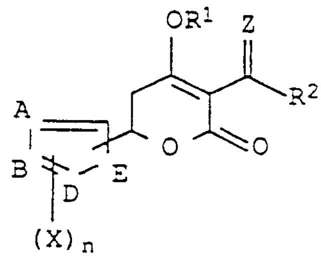

I

where $R^1$ is hydrogen;
$R^2$ is $C_1$-$C_6$-alkyl;
A, B and D are each CH or N, at least one of them being nitrogen;
E is oxygen, sulfur or -$NR^7$-;
$R^7$ is $C_1$-$C_6$-alkyl; benzyl or phenyl which may be monosubstituted to trisubstituted by halogen and/or $C_1$-$C_6$-haloalkyl,
X is halogen or $C_1$-$C_6$-alkyl or $C_3$-$C_6$-cycloalkyl which may be monosubstituted to trisubstituted by halogen, $C_1$-$C_6$-alkoxy and/or $C_1$-$C_6$-alkylthio; $C_2$-$C_6$-alkenyl; $C_2$-$C_6$-alkynyl; $C_1$-$C_6$-alkoxy; $C_2$-$C_6$-alkenyloxy; $C_1$-$C_6$-alkylthio; $C_1$-$C_6$-alkoxycarbonyl; $C_2$-$C_6$-alkanoyloxy; $NR^5R^6$; phenyl which may be monosubstituted to trisubstituted by halogen, nitro, cyano, $C_1$-$C_6$-alkyl, $C_1$-$C_6$-alkoxy, $C_1$-$C_6$-haloalkyl and/or $NR^3R^4$;
n is 0, 1 or 2, and, where n is 2, the radical X may be different;
$R^3$, $R^4$, $R^5$ and $R^6$ are each hydrogen or $C_1$-$C_6$-alkyl;
Z is oxygen or an oxime ether radical -$NOR^8$; and
$R^8$ is $C_1$-$C_6$-alkyl or $C_2$-$C_6$-alkenyl which may be monosubstituted to trisubstituted by halogen, $C_1$-$C_6$-alkoxy and/or $C_1$-$C_6$-alkylthio; $C_2$-$C_6$-alkynyl; $C_2$-$C_6$-haloalkynyl; $C_1$-$C_6$-alkyl or $C_2$-$C_6$-alkenyl, each of which may be monosubstituted to trisubstituted by phenyl, which may be monosubstituted to trisubstituted by halogen, nitro, cyano, $C_1$-$C_6$-alkyl, $C_1$-$C_6$-haloalkyl, $C_1$-$C_6$-alkoxy and/or $C_1$-$C_6$-alkylthio; or thenyl which may be monosubstituted to trisubstituted by halogen,
or an environmentally compatible salt thereof.

**2.** Tetrahydropyrandiones of the formula 1a

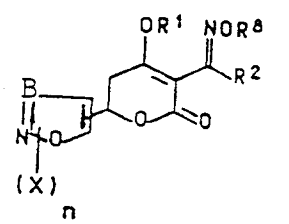

I a

where B, $R^1$, $R^2$, $R^8$, X and n have the meanings given in claim 1.

**3.** A herbicidal agent containing tetrahydropyran-2,4-dione of the formula I as claimed in claim 1, where Z is the radical -$NOR^8$.

**4.** A plant growth-regulating agent containing a tetrahydropyran-2,4-dione of the formula I as claimed in claim 1, where Z is oxygen.

**5.** A method for controlling undesirable plant growth wherein the undesirable plants and/or the area to be kept free from undesirable plant growth are treated with a herbicidally effective amount of a tetrahydropyran-2,4-dione of the formula I as claimed in claim 1, where Z is the radical -$NOR^8$.

**6.** A method for regulating plant growth, wherein an effective amount for growth regulation of a tetrahydropyran-2,4-dione of the formula I as claimed in claim 1, where Z is oxygen, is allowed to act

on the seeds, the plants and/or their habitat.

7. 3-[1-(cyclopent-2-enyloxyimino)-propyl]-6-[1-(3-trifluoromethylphenyl)-pyrrol-3-yl]-tetrahydropyran-2,4-dione.

8. A tetrahydropyran-2,4-dione as claimed in claim 1, wherein $R^1$, $R^2$, $R^7$, X, n and Z have the meanings given in claim 1 and the basic structure of the tetrahydropyran-2,4-dione, together with the heterocycle in the 6-position, forms one of the following structures I.1 to I.33.

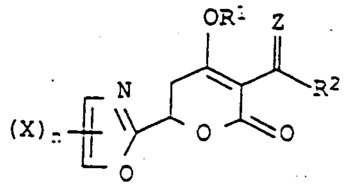

I.1

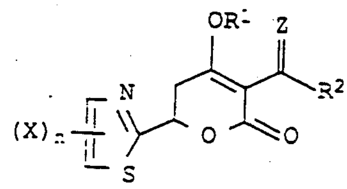

I.2

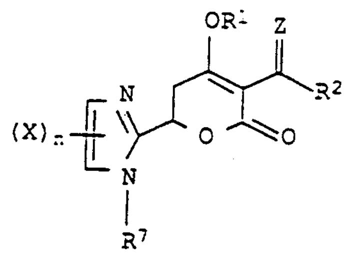

I.3

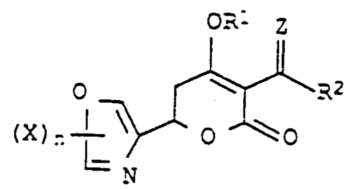

I.4

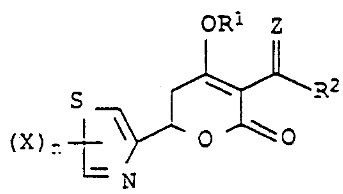

I.5

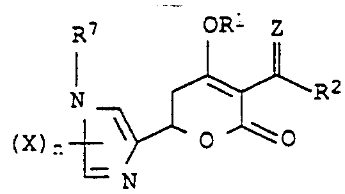

I.6

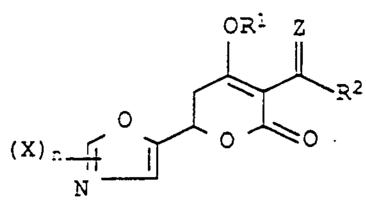

I.7

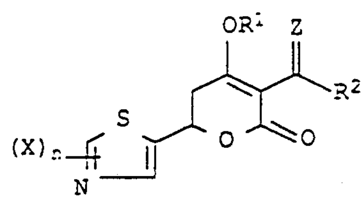

I.8

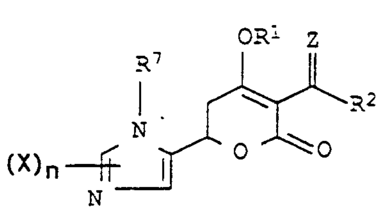

I.9

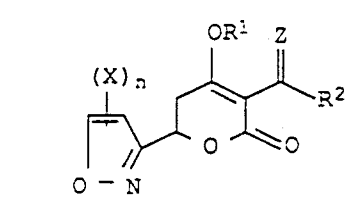

I.10

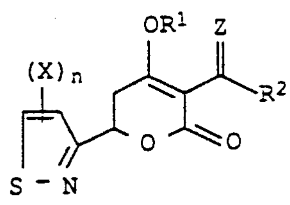

I.11

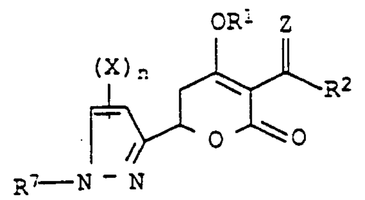

I.12

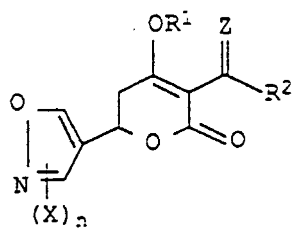

I.13

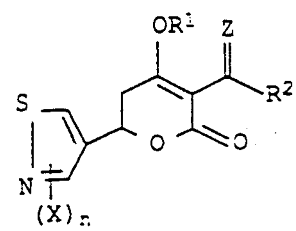

I.14

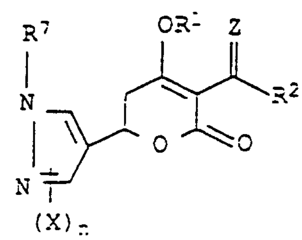

I.15

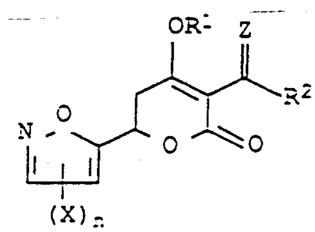

I.16

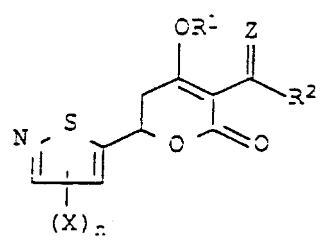

I.17

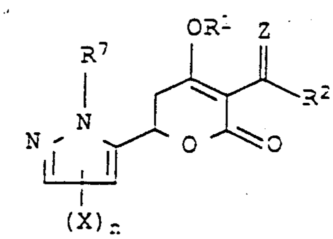

I.18

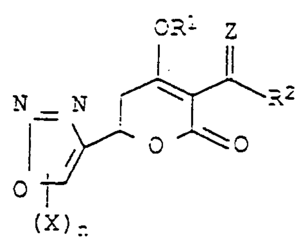

I.19

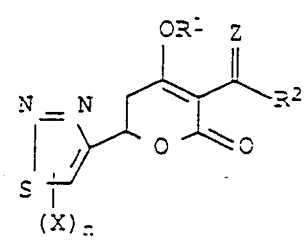

I.20

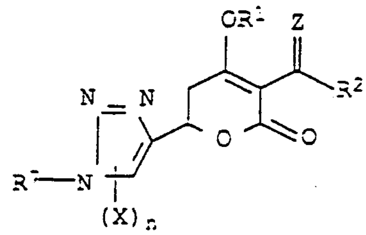

I.21

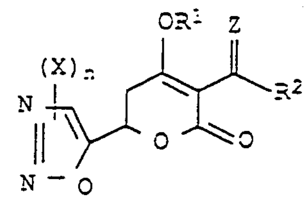

I.22

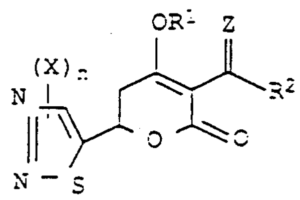

I.23

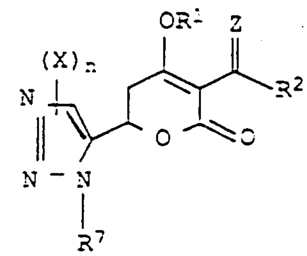

I.24

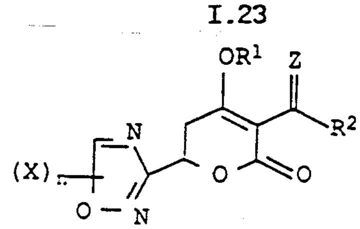

I.25

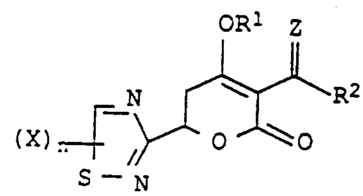

I.26

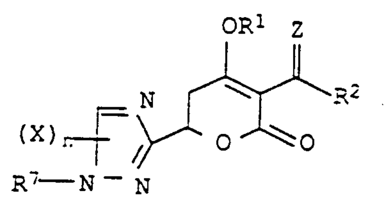

I.27

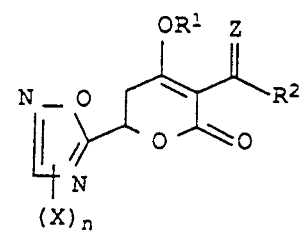

I.28

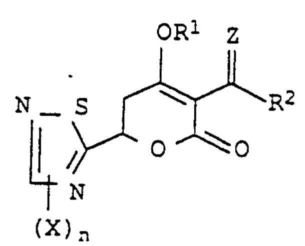

I.29

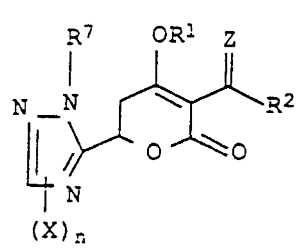

I.30

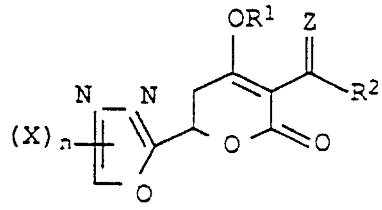

I.31

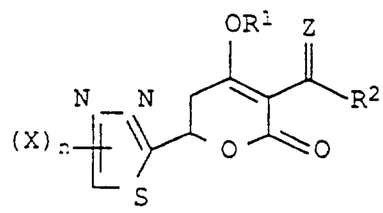

I.32

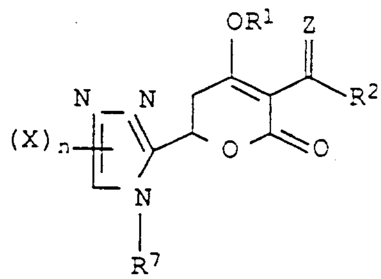

I.33

## Revendications

**1.** Tétrahydropyrane-2,4-diones de la formule I

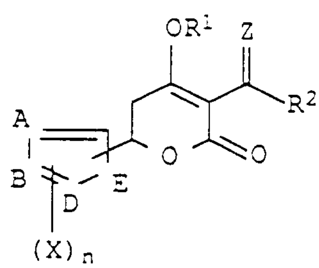

I

dans laquelle les substituants et les indices ont les significations suivantes :

$R^1$, hydrogène

$R^2$, alkyle en C1-C6,

A, B, D, CH ou N, au moins une des variables étant mise pour azote, E, oxygène, soufre ou $-NR^7-$,

$R^7$ étant mis pour alkyle en C1-C6, benzyle ou phényle qui peut être substitué une à trois fois par halogène et/ou halogénalkyle en C1-C6,

X, halogène, alkyle en C1-C6 ou cycloalkyle en C3-C6 qui peuvent être substitués une à trois fois par halogène, alcoxy en C1-C6 et/ou alkylthio en C1-C6, alcényle en C2-C6, alcinyle en C2-C6, alcoxy en C1-C6, alcényl en C2-C6-oxy, alkyl en C1-C6-thio, alcoxy en C1-C6-carbonyle, alcanoyle en C2-C6-oxy, $NR^5R^6$, phényle qui peut être substitué une à trois fois par halogène, nitro, cyano, alkyle en C1-C6, alcoxy en C1-C6, halogénalkyle en C1-C6 et/ou $NR^3R^4$,

n, 0, 1 ou 2, les restes X pouvant aussi être différents dans le cas où n = 2,

$R^3$, $R^4$, $R^5$, $R^6$ hydrogène, alkyle en C1-C6,

Z, oxygène ou un reste oximéther $-NOR^8$,

$R^8$ étant mis pour alkyle en C1-C6 ou alcényle en C2-C6, qui peuvent être substitués une à trois fois par halogène, alcoxy en C1-C6 et/ou alkyle en C1-C6-thio, alcinyle en C2-C6, halogènalcinyle en C2-C6, alkyle en C1-C6 ou alcényle en C2-C6, chacun substitué jusqu'à trois fois par phényle, qui peut être substitué une à trois fois par halogène, nitro, cyano, alkyle en C1-C6, halogènalkyle en C1-C6, alcoxy en C1-C6 et/ou alkyle en C1-C6-thio, phényle qui peut être substitué jusqu'à trois fois par halogène,

ainsi que leurs sels acceptables par l'environnement.

2. Tétrahydropyranediones de la formule Ia

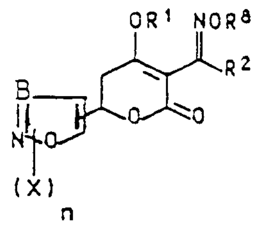

Ia

B, $R^1$, $R^2$, $R^8$, X et n ayant les significations données dans la revendication 1.

3. Agent herbicide, contenant une tétrahydropyrane-2,4-dione de la formule I selon la revendication 1, dans laquelle Z signifie le reste $-NOR^8$.

4. Agent régulateur de la croissance des plantes contenant une tétrahydropyrane-2,4-dione de la formule I selon la revendication 1, dans laquelle Z signifie l'oxygène.

5. Procédé pour lutter contre la croissance des plantes indésirables, caractérisé par le fait que l'on traite les plantes indésirables et/ou les surfaces à maintenir libres d'une croissance des plantes indésirables avec une quantité active au point de vue herbicide d'une tétrahydropyrane-2,4-dione de la formule I selon la revendication 1, dans laquelle Z signifie le reste $-NOR^8$.

6. Procédé pour régulariser la croissance des plantes, caractérisé par le fait que l'on fait agir sur les semences, les plantes et/ou leur biotope une quantité active pour la régulation d'une tétrahydropyrane-2,4-dione de la formule I selon la revendication 1, dans laquelle Z signifie oxygène.

7. 3-[1-(cyclopent-2-ényloxyimino)propyl]-6-[1-(3-trifluor-méthylphényl)pyrrol-3-yl]-tétrahydropyran-2,4-dione.

8. Tétrahydropyran-2,4-dione selon la revendication 1, $R^1$, $R^2$, $R^7$, X, n et Z ayant la signification donnée dans la revendication 1 et le corps de base de la tétrahydropyrane-2,4-dione formant, ensemble avec l'hétérocycle en position 6, une des structures I.1 à I.33.

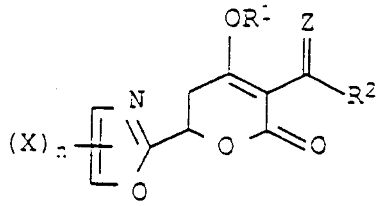

I.1

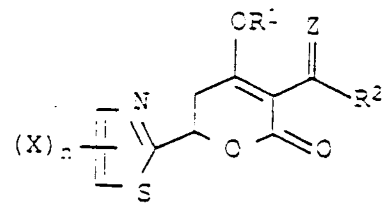

I.2

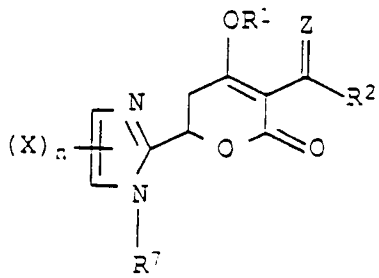

I.3

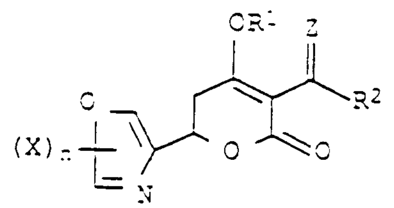

I.4

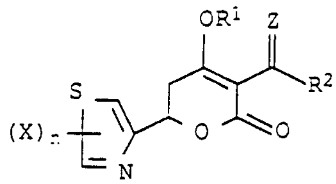

I.5

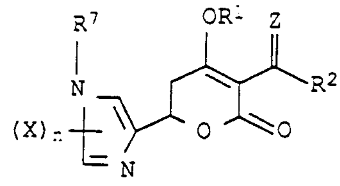

I.6

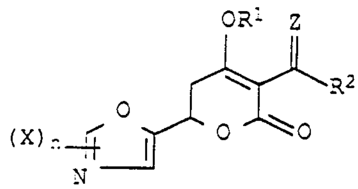

I.7

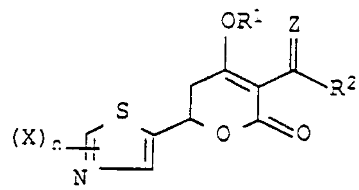

I.8

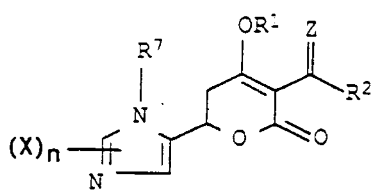

I.9

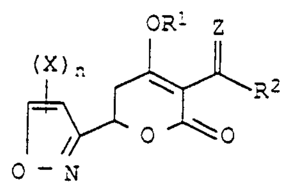

I.10

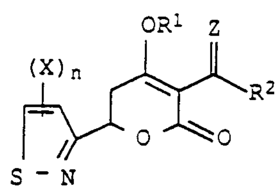

I.11

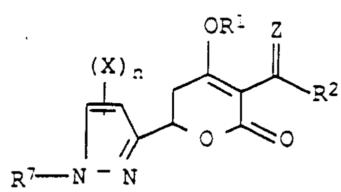

I.12

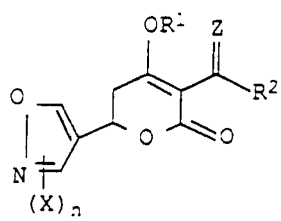

I.13

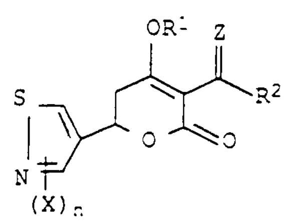

I.14

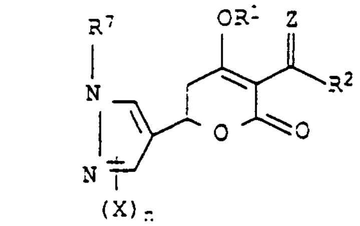

I.15

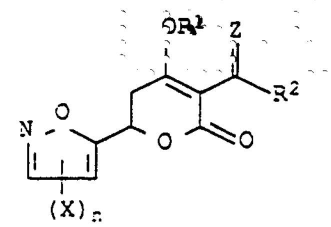

I.16

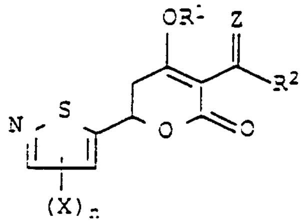

I.17

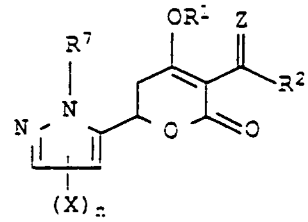

I.18

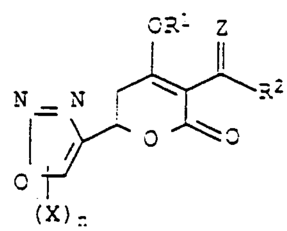

I.19

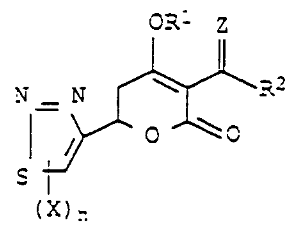

I.20

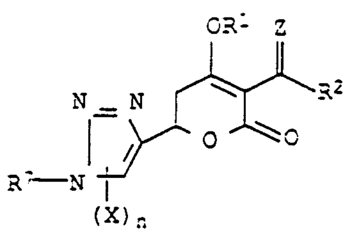

I.21

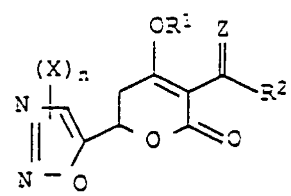

I.22

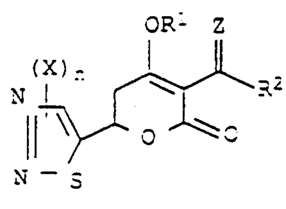

I.23

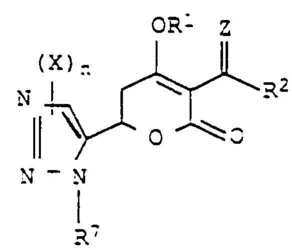

I.24

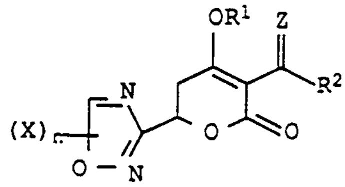

I.25

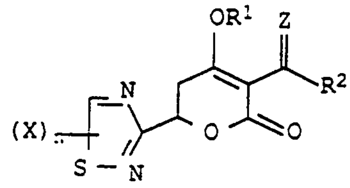

I.26

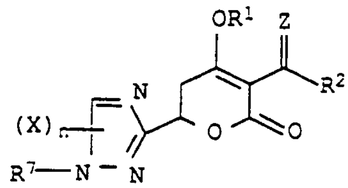

I.27

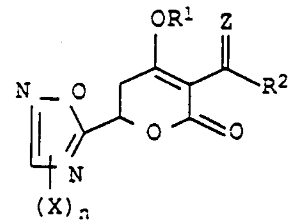

I.28

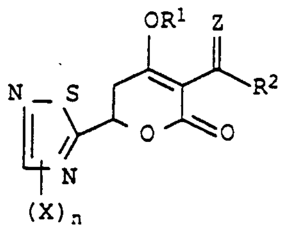

I.29

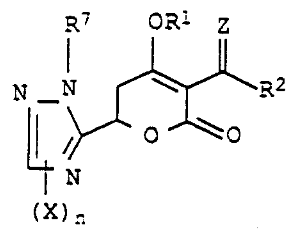

I.30

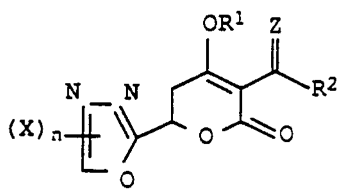

I.31

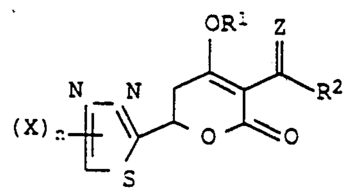

I.32

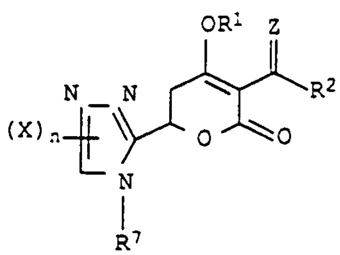

I.33